# EUROPEAN PATENT APPLICATION

(11) **EP 3 926 031 A1**
(43) Date of publication of application: **22.12.2021**
(21) Application number: 20180051.3
(22) Date of filing: 15.06.2020
(51) Int. Cl.: C12M 1/00, C12M 1/04, C12M 1/26, C12M 1/36, C12M 3/00, C12M 1/12

(54) **ASSEMBLY SYSTEM FOR A SMART TANK FOR A BIO-PHARMA PROCESS**

(71) Applicant: ESTR Biosystems GmbH, 37083 Göttingen (DE)
(72) Inventor: Schlack, Stefan, 37083 Göttingen (DE)
(74) Representative: Berger, Axel Bernhard

(57) **Abstract**

The present invention relates to an assembly system for assembling a modular smart tank, a method for assembling a modular smart tank, a mobile storage means and an assembly bag. The assembly system comprises at least one mobile storage means for storing components of at least one smart tank, an assembly room, wherein the assembly room is adapted to provide clean room conditions and wherein the assembly room is configured to allow assembly of at least one smart tank within the assembly room from the components stored in the mobile storage means, and a manipulator, being adapted to automatically assemble the smart tank within the assembly room.

## Description

### Field of the Invention

The present invention relates to an assembly system for assembling a modular smart tank, a method for assembling a modular smart tank, a mobile storage means and an assembly bag. The assembled smart tank and/or a system comprising multiple assembled smart tanks may be used in a bio-pharma process line. Further, the smart tank and/or smart tank system may be adapted for manufacturing and/or developing e.g. bio-pharmaceutical goods and/or for developing and/or testing the commercial manufacturing process of the same. The assembly system, the method for assembling a modular smart tank, the mobile storage means and/or the assembly bag allow for an automated assembling of smart tank(s) under clean room conditions and for an improved sterilization of the same.

### Background Art

Bio-pharmaceutical goods, such as drugs, e.g. for cancer therapy, genetic therapy and/or cell therapy, are nowadays manufactured in so called bio-pharma process lines.

Known bio-pharma process lines are e.g. based on stainless-steel tanks that are interconnected by pipes and/or the like. Those known bio-pharma process lines are difficult to maintain and to clean. Thus, so called single-use bio-pharma process lines were developed that are replaced after usage. Here, single-use containers, such as bags, serve as reservoirs for educts and/or products of the bio-pharma process line. Said bags are typically supported in rigid containers, such as stain-less steel containers, and are interconnected by hoses. Setting up a conventional single-use bio-pharma process line is very complex and time consuming, as manual assembly is required. In particular, the components of the single-use bio-pharma process line have to be manufactured and pre-assembled. Then, the single-use bio-pharma process line has to be installed/assembled at an operator's site.

For example, multiple bags, filters, sensors, valves, etc., have to be connected via various hoses. Manual pre-assembly of a conventional single-use bio-reactor tank takes about up to one day. Thus, setting-up conventional single-use bio-pharma process lines is very expensive and time consuming. Thus, in case large amounts of a specific drug are needed immediately, e.g. due to an pandemic, long set-up times may lead to drug shortage and thus live is endangered. Further, expensive components such as sensors typically cannot be re-used and have to be discarded with the single-use bio-pharma process line, after use. Still further, the complex assembly bears a significant risk for mal-function of the bio-pharma process line, due to improper or incorrect assembling.

Still further, the single-use bio-pharma process lines a prone to damages as the (hosebased) connections and/or bags of the single use containers tend to leakages over the operational time. In case of leakage or mal-function, the educts and/or products of the entire bio-pharma process line can be damaged or even destroyed. Accordingly, there is a significant economic risk for the operator of the bio-pharma process line.

The bio-pharma process line, the tanks and/or the tank system thereof have to be sterilized prior to usage. Typically gamma sterilization is used. However, gamma sterilization negatively affects the components of the single-use bio-pharma process line, in particular plastic components. Further, gamma sterilization has to be carried out, using a gamma source. These gamma sources are limited in capacity so that sterilization is a bottle neck in known single-use bio-pharma process lines.

After sterilization, the tanks or the tank system is transported to a manufacturer of biopharmaceutical goods for manufacturing bio-pharmaceutical goods. Those sterilized single-use bio-pharma process lines, tanks or tank systems have an expiration date of about 6 to 36 months, depending on the components. This is, as the sterilization is not durable. Accordingly, after the expiration of about 6 to 36 months, the tanks/tank system has to be sterilized anew. Further, a single-use bio-pharma process line is typically output-specific. I.e. only a single product can be manufactured with the specific single-use bio-pharma process line. If, e.g. the sells of the product remain under expectation, expensively sterilized single-use bio-pharma process line cannot be used otherwise.

Further, after usage, all components of the single-use bio-pharma process line are must be discarded, as disassembly, cleaning and/or recycling is impossible.

In view of the above, it is an object of the present invention to provide an assembly system for assembling a modular smart tank, a method for assembling a modular smart tank, a mobile storage means and an assembly bag that overcome the above-mentioned drawbacks. Further, costs and time required for developing and manufacturing biopharmaceutical goods shall be reduced, thereby allowing a faster time-to-market.

### Summary of the Invention

The object is achieved by an assembly system for assembling a modular smart tank, a method for assembling a modular smart tank, a mobile storage means and an assembly bag. Further embodiments are described in the dependent claims and the following description. Parts of the description that do not fall under the scope of the claims are provided to give a better understanding of the claimed invention.

In particular, the object is achieved by an assembly system for assembling a modular smart tank for a bio-pharma process under clean room conditions. The system comprises at least one mobile storage means for storing components of at least one smart tank. The system comprises further an assembly room, wherein the assembly room is adapted to provide clean room conditions and wherein the assembly room is configured to allow assembly of at least one smart tank within the assembly room from the components stored in the mobile storage means. Further, the system comprises a manipulator, being adapted to automatically assemble the smart tank within the assembly room.

The assembly system serves for an automated assembly of at least one smart tank from the components stored in the mobile storage means. In the mobile storage means, all components required for assembling the smart tank may be provided. Alternatively, the components required for assembling the smart tank may be distributed over several mobile storage means. For example, the elements, such as a top plate element, at least one sidewall element and/or a bottom plate element, that form the reservoir of the smart tank may be provided in a first mobile storage means. Additional components, such as valves, filters, sensors and/or the like may be provided in a second mobile storage means.

The mobile storage means can for example be equipped at a manufacturer site with the components of the smart tank. Subsequently, the container, and thus the components stored therein, can then be sterilized. This may be carried out using ETO or by autoclaving. Gamma sterilization is also possible. As the smart tank is disassembled, i.e. provided in components, when being stored in the mobile storage means, sterilization is facilitated. This is, as inner lumens, such as the reservoir of the smart tank, is not yet built and thus, all surfaces that will e.g. form the reservoir when the smart tank is assembled can easily be reached and sterilized.

Sterilization can be carried out at the manufacturer site or at the manufacturer of the bio-pharmaceutical goods. Sterilization maybe automated in that the mobile storage means are automatically guided to a sterilization station, that is adapted to sterilize the mobile storage means. In particular, the mobile storage means may comprise a port that serves as an inlet port for ETO gas and/or heated steam. Thus, the mobile storage means and the components stored therein can be delivered and stored until use. Only just before assembly and use, the components can be sterilized within the mobile storage means. The port may be covered by a filter, e.g. a sterile filter, that allows to provide operating medium into the mobile storage means sterile. After sterilization, the mobile storage means may be filled with a sterile inert gas and/or the like. As will be described later, the port and optionally the filter allow for integrity testing of the mobile storage means. For transport purposes, the port/filter may be covered be a cover means, such as a cap.

By sterilizing components of a smart tank, preferably within a mobile storage means, instead of sterilizing assembled smart tanks, the time required for sterilization can be reduced. This is, as only very few assembled smart tanks can be loaded into e.g. an autoclave. In a disassembled state the packing size is smaller and more smart tanks (respectively components of a smart tank) can be loaded into the autoclave. Thus, less sterilization cylces are needed. In case the components are stored within the mobile storage means for sterilization, the process can even be speeded up. As the components are then assembled under clean room or even sterile conditions, the assembled smart tanks need not to be sterilized anew.

The mobile storage means may be a multi-use device, or the mobile storage means may be provided as single use device. In case of a multi-use device, the mobile storage means may be made substantially of stainless steel. Thus, a durable and rigid mobile storage means can be provided. In case of a single use device, the mobile storage means may be a plastic-based device that can be discarded after use.

The assembly room allows assembling of at least one smart tank within the assembly room from the components stored in the mobile storage means. Particularly, the assembly room provides at least clean room conditions. The assembly room may provide at least ISO 8 (ISO 14644-1 and ISO 14698) conditions. Depending on the required environment, clean room conditions up to ISO 1 are also possible. More preferably, the assembly room provides a sterile environment for assembling the smart tank(s). The assembly room may be sterilizable, e.g. by ETO or autoclaving.

For assembling the smart tank within the assembly room, the components may be transferred separately to the assembly room. Particularly, the assembly room may be adapted to sealingly couple to at least one mobile storage means. Thus, components can be removed from the mobile storage means and can be assembled within the assembly room to form a smart tank. Alternatively, or additionally, at least one mobile storage means may be provided within the assembly room. Thus, components that are used in several different kinds of smart tanks, such as filters, valves, sensors, or the like, can be provided individually.

In case the assembly room is sealingly coupled to the mobile storage means, the sterilized components stored in said mobile storage means can be easily transferred to the assembly room, without risking contaminating the sterilized components.

The manipulator may be an industrial robot that allows manufacturing and assembling the at least one smart tank from the components stored in the mobile storage means. The manipulator may comprise multiple industrial robots that can be operated together. Thus, complex assembling tasks can be carried out. For example, a first industrial robot may grip a first component from the mobile storage means and a second robot may assemble a second component to the first. As an industrial robot typically provides several degrees of freedom (e.g. 6), the first component can be held under different orientations. Accordingly, the second components may be assembled on almost any portion of the first component. Further, the manipulator may be equipped with different kinds of actuators. These actuators, such as different gripping tools, assembling tools (e.g. screw drivers, welding tools,...) or disassembling tools, may be interchangeable. The interchangeable actuators may be provided within the assembly room. Thus, an automated and flexibel assembling system can be provided that is adapted to assemble different kinds of smart tanks, smart tank systems and/or process lines.

The mobile storage means may include a sealable opening that is configured for allowing removal of components of the at least one smart tank from the mobile storage means. Likewise, the assembly room may comprise at least one corresponding sealable opening that is configured for receiving components from a mobile storage means. Thus, components can be transferred from the mobile storage means to the assembly room. The risk of contamination is thereby reduced significantly.

Further, the sealable opening of the mobile storage means and the corresponding sealable opening of the assembly room may be configured to open jointly, when the assembly room is sealingly coupled to the mobile storage means. Thus, transferring components from the mobile storage means to the assembly room is facilitated.

The sealable opening of the mobile storage means may be covered by at least one cover element. The corresponding sealable opening of the assembly room may be covered by at least one corresponding cover element. The at least one cover element and the at least one corresponding cover element are configured to couple with each other to open jointly.

For example, the sealable opening and the corresponding sealable opening may be provided as sliding doors. The at least one cover element/corresponding cover element maybe a door leaf of the respective sliding door. Coupling the cover element(s) with the corresponding cover element(s) can be achieved e.g. by magnetic means, positive locking means, or any other coupling or a locking means. Preferably, magnets (permanent of electric) are distributed at least on an edge portion of the cover element(s)/corresponding cover element(s). This allows coupling the cover element(s) with corresponding cover element(s) in a defined manner. Thus, the non-sterile outer faces of the cover element(s)/corresponding cover element(s) cover each other after coupling. Thus, the risk for contaminating the components when being transferred through the sealable openings form the mobile storage means to the assembly room can be further reduced.

The cover element(s)/corresponding cover element(s) are movable with respect to the mobile storage means and/or the assembly room. The cover element(s)/corresponding cover element(s) and the mobile storage means/assembly room may be sealed, e.g. by at least one brush seal, a lip seal and/or the like. Even more preferably, the cover element(s)/corresponding cover element(s) may be connected with the mobile storage means, or the assembly room via a flexibel portion, such as a sealing foil portion. The flexibel portion may be connected to the cover element on a first side and to the mobile storage means on a second side. Likewise, the flexibel portion may be connected to the corresponding cover element on a first side and to the assembly room on a second side. Thus, the sealable opening can be opened/closed without providing a gap between the cover element and the mobile storage means and/or the corresponding cover element and the assembly room, respectively. Thus, the contamination risk can be further reduced.

The system may further comprise a sealing frame. The sealing frame may be configured to frame the sealable opening of the mobile storage means and/or the corresponding sealable opening of the assembly room. Thus, an additional seal is provided that seals the opening from the environment, when being opened. Thus, the contamination risk can be further reduced. The sealing frame may be a separate frame, may be provided on the mobile storage means, and/or the assembly room.

The system may further comprise a UV-light source. The UV-light source may be associated with the sealable opening of the mobile storage means and/or the corresponding sealable opening of the assembly room. Thus, possible contaminants on the outer surface of the sealable opening can be removed/destroyed prior to and/or during opening the sealable openings. The at least one UV-light source may be provided within the sealing frame. Preferably, the UV-light source is configured to illuminate the sealable opening(s) from different orientations. Thus, contaminants can be removed/destroyed effectively.

The sealing frame may further comprise a port that allows evacuating a space between the sealing frame and the closed sealable openings of the mobile storage means and/or the assembly room prior to opening the sealable openings. Thus, particles and/or contaminants can be effectively removed and the risk of contamination can be further reduced.

The at least one manipulator may be adapted to remove components from the mobile storage means and to assemble a smart tank within the assembly room from the removed components to form an assembled smart tank. Optionally the manipulator is further adapted to assemble at least one of the following, a filter, a valve and/or a sensor, to the assembled smart tank. Thus, different kinds of smart tanks can be assembled allowing for a flexible assembling system.

Further, the manipulator may be adapted to position at least one (smaller) smart tank on top of an assembled smart tank. This small smart tank may be filled with supplements, additives, acids, bases, and/or the like, that are required for operating the biopharmaceutical process line. Alternatively, the at least one (smaller) smart tank may be positioned on top of the assembled smart tank at a later point in time. In particular, the at least one (smaller) smart tank may be provided in a non-sterile (non-clean room) environment wherein the assembled, larger smart tank is placed in a sterile or clean room environment. A barrier may separate the non-sterile (non-clean room) environment from the sterile or clean room environment, wherein the barrier (such as a foil portion) is sandwiched between the at least one (smaller) smart tank and the assembled smart tank. According to this alternative, the at least one (smaller) smart tank may be positioned on top of an assembled smart tank manually and/or via a handling manipulator. Both smart tanks may be fluidically connected, via a channel or port. The channel or port may comprise a sterile filter, allowing to transfer medium from the smaller smart tank to the larger smart tank without contaminating the larger smart tank. The fluid connection may be controlled by a valve. The sterile filter may be arranged at least partially in a top plate element of the larger smart tank.

The system may include components of at least one modular smart tank, the components include a top plate element, at least one sidewall element, and a bottom plate element. The top plate element, the at least one sidewall element and the bottom plate element can be assembled to form a reservoir of the smart tank for receiving at least one biochemical medium. At least one of the top plate element, the at least one sidewall element and/or the bottom plate element comprises at least one channel, for guiding the at least one biochemical medium and/or an operating medium.

Generally, all channels and/or reservoirs of the smart tank may be configured to be self-emptying. I.e. medium that has entered the channel and/or reservoir can flow out of the respective channel and/or reservoir by gravitation.

The assembled smart tank may comprise one reservoir or multiple (at least two) reservoirs, formed by the top plate element, at least one sidewall element, and a bottom plate element. In case of multiple reservoirs, the reservoirs may be arranged serial or parallel to each other. Medium flowing through serial reservoirs flows through the single reservoirs one after another. Medium flowing through parallel reservoirs flows through the single reservoirs in a parallel fashion. Combinations of serial and parallel arrangement of the reservoirs is also possible.

The assembled smart tank is then adapted for being used in a bio-pharma process line, for manufacturing and/or developing bio-pharmaceutical goods and/or for developing and/or testing the commercial manufacturing process of these goods. Further, the smart tank may be used in other manufacturing lines and may be adapted to be operated automatically and/or manually.

The biochemical medium and/or the operating medium may be any educt or product of the process line, such as sample containing medium, cell containing medium, drug containing medium, buffer medium, acids, bases, and/or the like. The medium may comprise at least one of the following: small molecule API, antibody, drug conjugates, RNA or fragments thereof, rec proteins, viral vaccines, bacterial/microbial processes, virus like particles, viral vectors, ADC, DNA, and/or the like.. Further, the operating medium may comprise heating or cooling fluids, pressurized air or gases, and/or the like. For example, in case pressurized air is used as operating medium, the operating medium may serve for driving a biochemical medium through the channel and/or into (or out of) the reservoir of the smart tank.

The biochemical medium and/or the operating medium may be provided in liquid and/or gaseous form as well as in form of solutions and/or emulsions and/or suspensions. The biochemical medium and/or operating medium can be filled to the assembled smart tank at the assembling room, or at a different location.

The terms top plate element, side wall element and bottom plate element do not specify the orientation of the smart tank, when being assembled and in use. Rather, the assembled smart tank may be used in any orientation. Typically, the bottom plate element serves as a base and can optionally be provided on a stand for adjusting the height of the smart tank, when being in use/operation. For example, the orientation of the smart tank can be a standing orientation (i.e. the top plate element is on top), a lying orientation (i.e. a side wall element is on top), or an upside-down orientation (bottom plate element is on top).

Providing a top plate element, at least one side wall element and bottom plate element that are arranged to form a reservoir for receiving at least one biochemical medium facilitates cleaning and maintaining the smart tank after being used. Thus, the elements or at least some of the elements can be reused and/or recycled separately. Further, the smart tank - respectively the elements forming the reservoir - can be easily cleaned and sterilized prior to use. Optionally, the at least one side wall element may be integrally formed with the top plate element or the bottom plate element. Thus, assembling the smart tank is facilitated.

The at least one channel of the smart tank is configured for guiding at least one biochemical medium and/or an operating medium. Optionally, the smart tank may comprise multiple channels, wherein each of said channels may be adapted to guide a different biochemical medium and/or an operating medium, if the smart tank is in use. The at least one channel extends within at least one of the top plate element, the at least one sidewall element and the bottom plate element. Different channels may be provided in different or same elements, so that each of the top plate element, the at least one sidewall element and/or the bottom plate element may comprise at least one (or multiple) channels.

A channel is any inner lumen that extends in the top plate element, the at least one sidewall element and/or the bottom plate element and that is adapted to guide a flow of a biochemical medium and/or an operating medium. Particularly, the channel may be integrally formed with the respective element(s). The channel may be in communication with the reservoir of the smart tank and/or may bypass the reservoir of the smart tank.

The channel may have a diameter in the range of o.i to 3 inch (0.25 to 7.6 cm), preferably in the range of 0.2 to 2 inch (0.5 to 5.1 cm), more preferably in the range of 0.3 to 1.5 inch (0.75 to 3.8 cm), even more preferably in the range of 0.5 to 1 inch (1.2 to 2.5 cm) and most preferably about 0.75 inch (1.9 cm). Further, different channels may have different diameters and/or the diameter of the channel may vary. Thus, e.g. nozzles may be provided.

Particularly, a channel may extend, at least partially, in the top plate element, the at least one sidewall element and/or the bottom plate element, wherein the length of the channel is longer than the thickness of the respective top plate element, sidewall element and/or the bottom plate element. Accordingly, the medium may be guided by the channel in a direction that is different from a surface normal of a main surface of the respective top plate element, sidewall element and/or the bottom plate element.

Generally, the smart tank may serve as reservoir for any educt or product of the process line, such as a biochemical medium and/or an operating medium. Thus, the received medium may be stored and/or transported. Further, the smart tank may be configured to blend different mediums received in the reservoir of the smart tank. Further, the smart tank may serve as bioreactor and may support a biologically active environment. For example, a chemical process which involves organisms or biochemically active substances derived from such organisms can be carried out in said smart tank. Further, the smart tank may be designed to grow cells or tissues in the context of a cell culture. The smart tank may be used as a batch bioreactor, a concentrated fed batch bioreactor, a fed batch bioreactor or a continuous bioreactor and/or a perfusion bioreactor. Additionally, or optionally, the smart tank may serve as filtration unit for filtering the medium received in the reservoir of the smart tank.

The reservoir of the smart tank may have a volume of at least about 10 ml, of at least about 15 ml, of at least about 20 ml, of at least about 50 ml, of at least about 100 ml, of at least about 200 ml, of at least about 250 ml, of at least about 500 ml, of at least about 1 l, of at least about 2 l, of at least about 5 l, of at least about 10 l, of at least about 20 l, of at least about 50 l, of at least about 100 l, of at least about 200 l, of at least about 500 l, of at least about 1000 l, of at least about 2000 l, of at least about 3000 l, of at least about 4000 l, or of at least about 5000 l. Thus, bio-pharma process lines can be scaled. For example, during development smart tanks with a small volume are used. Afterwards, during manufacturing, larger smart tanks are used. Further, smart tanks of different volumes can be combined in a smart tank system of a bio-pharma process line.

The smart tank may have a height dimension, a depths dimension and a widths dimension, wherein the ratio of hight: depth:width may be of about 2:1:1. This ratio has to be shown to be preferred in case the smart tank is used as a bioreactor. Other dimensions maybe chosen, depending on the desired functionality of the smart tank. For example, it is possible to provide different kinds of sidewall elements. The side wall elements may have a width in the range of 80 mm to 1500 mm, preferably in the range of 200 mm to 1200 mm, even more preferably in the range of 300 mm to 1000 mm and most preferably in the range of 450 mm to 600 mm. Further the side wall elements may have a height in the range of 150 mm to 2000 mm, preferably in the range of 200 mm to 1300 mm, even more preferably in the range of 500 mm to 1000 mm and most preferably in the range of 450 mm to 650 mm. Thus, different dimensions and volumes can be provided, using the same top plate element and bottom plate elements. Further, different kind of top plate elements and bottom plate elements can be combined with the same kind of sidewall elements. Particularly, the manipulator may assemble respective elements to form different kinds of assembled smart tanks.

In particular, the smart tank maybe a multi-functional smart tank comprising multiple channels of different channel-types and/or the same channel type. At least one channel may be opened and closed, using e.g. a valve, a closure, or the like. Thus, depending on desired functionality a channel or multiple channels can be closed. These closed channels are not used. At least one other or multiple other channels may be opened and therefore used, thereby defining the functionality of the multi-functional smart tank. The at least one valve may be assembled by the manipulator.

The smart tank may comprise at least one port, wherein the at least one port may be associated with a respective channel. The port may be chosen from a group of port-types, comprising the following port-types: a fluid inlet port, a gas inlet port, a fluid outlet port, a gas outlet port, a cell blead port, a tank-interconnecting port, an element-interconnecting port, a medium supply port, a medium remove port and/or the like.

The (fluid or gas) inlet port serves for providing a biochemical medium and/or an operating medium to the reservoir of the smart tank. A (fluid or gas) outlet port serves for removing a biochemical medium and/or an operating medium from the reservoir of the smart tank. A medium supply port allows to supply medium (biochemical medium and/or an operating medium) to the smart tank and a medium remove port allows to remove medium (or parts thereof) from the smart tank. The medium remove port may also server for transferring a retentate from e.g. a filter or a membrane back into the smart tank. A tank-interconnecting port serves for coupling a channel of a first smart tank fluidically to a corresponding channel of a second smart tank, when the first smart tank is connected with the second smart tank, e.g. by using a connector means. A tank-interconnecting port may serve as medium supply port and/or medium remove port. A cell blead port serves for removing cells from the reservoir. The cells may either be discarded or may be transferred to a further smart tank or other device for further processing.

For example, a channel may be associated with a tank-interconnecting port and a fluid outlet port. Thus, said channel may serve for transferring a medium, such as a fluid, from the reservoir of the smart tank to a further smart tank.

The at least one port may comprise a protruding shroud that at least partially surrounds the associated channel. The shroud may be concentrically arranged around a channel end of the associated channel. Further, the at least one port may comprise a recess, that at least partially surrounds the associated channel. The recess may be concentrically arranged around a channel end of the associated channel. Said port may be adapted to be coupled to a hose element or a pipe element so as fluidically connect the smart tank with further objects in the periphery. For example, a smart tank may be integrated in a conventional bio-pharma process line. The connection of the smart tank with further objects in the periphery may be performed using the manipulator. Particularly, the smart tank may comprise a triclamp adaptor, a tube clip, a quick connector, and/or the like for connecting the smart tank with further objects in the periphery.

Further, the port (e.g. a tank-interconnecting port or an element-interconnecting port) may be adapted to engage with a corresponding port (e.g. a corresponding tank-interconnecting port or an element-interconnecting port), wherein the port and the corresponding port are formed to provide a positive locking. For example, the smart tank may comprise a port on a first side and a corresponding port on a second side, wherein the first side and the second side may be opposing outer surfaces of the smart tank. This allows to couple the smart tank with a further smart tank using the port and the corresponding port and to fluidically connect the respective channels when coupling the smart tanks. Thus, a medium can be transferred from a first smart tank to a second smart tank without the use of intermediate conduit elements, such as pipes or hoses. Thereby assembling a smart tank system of e.g. a bio-pharma process line is facilitated and less prone to assembly mistakes. In particular, assembling a smart tank system by interconnecting different smart tanks can be achieved, using the manipulator.

Further, an element of the smart tank (top plate element, sidewall element and/or bottom plate element) may comprise a port. A further element of the smart tank (top plate element, sidewall element and/or bottom plate element) may comprise a corresponding port. This allows to couple the elements to form the reservoir of the smart tank and thereby to fluidically connect the respective channels of the elements when assembling the smart tank. Thus, a medium can be guided though a channel formed in at least two elements of the smart tank. Thereby assembling a smart tank using the manipulator is facilitated and less prone to assembly mistakes, compared to a manual assembly.

The ports may include a sealing member (e.g. flexible sealing member, such as a rubber seal, a silicon seal, a Teflon seal, or the like). Said sealing member may be a radial and/or an axial sealing. For example, the sealing member may be provided on a shroud and/or within a recess of the port. The sealing member maybe provided as sealing gasket that allows to seal multiple ports of the smart tank. Further, the sealing member may be port specific and adapted to seal only a single port.

The smart tank may comprise multiple tank-interconnecting ports to provide an interconnecting interface that allows to easily couple the smart tank to a further smart tank. Further, each element of the smart tank may comprise multiple element-interconnecting ports to provide an interconnecting interface that allows to easily couple different element of the smart tank to assemble the smart tank.

The smart tank may comprise at least one filter, wherein the least one filter may be chosen from a group of filter-types, comprising the following filter-types: a pre-filter, a sterile filter, a bacterial filter, a viral filter, a mycoplasma filter, an ultrafiltration filter, a diafiltration filter, a cell filter, a fluid filter, an air filter, a gas filter, or the like. The filter may be provided within the reservoir of the smart tank. Thus, the smart tank may serve as filtration unit for filtering the medium received in the reservoir of the smart tank. The at least one filter may be assembled to the smart tank using the manipulator.

The smart tank may comprise at least one valve. The at least one valve may be associated with the at least one channel. The at least one valve may be a flow control valve, a cutoff valve, a pressure relief valve or a non-return valve, or the like. Further, a smart tank may comprise multiple valves of the same and/or of different types. Particularly, a valve may be provided at a junction of at least two channel portions. Thus, depending on the valve position, medium can be guided to different channels and/or tanks.

The at least one valve, a valve body thereof and/or actuating means for actuating the valve may be initially integrated in the respective element of the smart tank or may be adapted to be integrated in the respective element of the smart tank after or during assembly of the smart tank. The at least one valve, valve body thereof and/or actuating means may be assembled to the smart tank using the manipulator. This facilitates the assembly of the smart tank and/or a smart tank system of a bio-pharma process lines.

The smart tank may further comprise at least one connector means for interconnecting the smart tank (first smart tank) with a further (second) smart tank. The connector means allows a fast set up of a smart tank system, comprising several smart tanks, as the smart tanks can be interconnected by said connector means. Thus, the assembly of a bio-pharma process line is facilitated and/or speeded up. Particularly, assembly of the bio-pharma process line can be done, using the manipulator. Due to the automated assembling process, the risk of mal-function and/or leakage due to improper assembly can be reduced.

The connector means may be threaded connector means such as screws and respective nuts and/or belts or straps that interconnect the first and second smart tank. The connector means may be a latching connector means, wherein the smart tank comprises a first latching connector means for directly interconnecting the smart tank with a further smart tank, which comprises a corresponding latching connector means. The first latching connector means may be a latching arm including a latching protrusion. The latching protrusion may be adapted to latch with a corresponding latching connector means, which maybe formed as latching recess.

The smart tank may alternatively or additionally comprise a second latching connector means, which is configured to latch with an inter-latching connector means, that is adapted to latch with a second latching connector means of a further smart tank, so that the smart tank can be directly interconnected with said further smart tank, via the inter-latching connector means. Said second latching connector means may include a protrusion, such as a nob, that is adapted to latch with an inter-latching connector means, having e.g. a corresponding recess to provide a positive locking. The inter-latching connector means may be a flexible inter-latching connector means, that is tensioned upon latching with the second latching connector means of the smart tank(s), thereby urging a first smart tank against a second smart tank.

The connector means may provide a fluidical connection between the first and second smart tank. For example, the connector means may be designed and shaped, so that upon latching tank-interconnecting ports of the first and the second smart tanks engage which each other so that respective channels are aligned and in (sealed) fluid communication. Thus, the assembly of a smart tank system of a bio-pharma process line can be facilitated and speeded up.

The connector means and in particular the latching connector means may be provided at the top plate element, the at least one side wall element and/or the bottom plate element. Further, the connector means may be provided in a recessed portion of top plate element, the at least one side wall element and/or the bottom plate element so as to prevent the connector means from being damaged during transport and/or storage.

Particularly, the connector means may be configured to be operated automatically using the manipulator and/or tool less. The connector means may provide a permanent interconnection of the smart tanks or a separable interconnection.

The top plate element, the at least one sidewall element and/or the bottom plate element may be formed from a plastic material. For example, the elements may be formed from COC (cyclic olefin copolymer), COP (cyclic olefin polymer), PP (polypropylene), PC (poly carbonate), PET (polyethylene terephthalate), and/or the like.

The top plate element, at least one of the sidewall elements and/or the bottom plate element of the smart tank may comprise at least one assembly-connecting means and/or at least one corresponding assembly-connecting means. The assembly-connecting means and the corresponding assembly-connecting means are configured to engage with each other, so as to secure an assembly of at least two adjacent elements, chosen from the group of top plate element, one or more sidewall elements and bottom plate element.

For example, the assembly-connecting means may be a threaded member that is integrated into at least one of the elements. The threaded member may have an internal thread or an external thread and may be integrally formed with the respective element. Optionally or additionally, the threaded member may be an inlay, such as a metal inlay, that is securely held in the respective element of the smart tank. An inlay can for example be overmolded or glued into the respective element. The corresponding assembly-connecting means can be a trough opening that can be aligned with the threaded member having an inner thread. Thus, the elements can be connected and engaged by threading a screw through the through opening into the threaded member. This can be done automatically, using the manipulator. Further, the corresponding assembly-connecting means can be a trough opening that receives a threaded member having an outer thread. Thus, the elements can be connected and engaged by threading a nut and or the like on the threaded member, thereby engaging the elements. This can be done automatically, using the manipulator.

Further, the assembly-connecting means may be protrusions, such as bolts. The protrusions can be integrally formed with the respective element or can be an inlay. The inlay can for example be overmolded or glued into the respective element. The corresponding assembly-connecting means may be a corresponding recess. The recess may be integrally formed with the respective element or can be an inlay, such as a sleeve. The inlay can for example be overmolded or glued into the respective element. During assembly, the assembly-connecting means engage with the corresponding assembly-connecting means and provide a positive locking. The engagement of the assembly-connecting means and the corresponding assembly-connecting means may be a self-retaining engagement. The self-retaining engagement may be achieved by a retaining force provided by a flexible member that may be provided between the elements that are engaged (e.g. a top plate element and a sidewall element, two different sidewall elements and/or a bottom plate element and a sidewall element). Due to assembly, the flexible member maybe compressed, thereby providing a retaining force. Particularly, the flexible member may be a sealing member that seals the reservoir formed by the elements.

The smart tank may comprise at least one sealing member for providing a sealed connection between the element (top plate element, at least one sidewall element and bottom plate element). The sealing member may be arranged circumferentially at each sidewall element, top plate element and/or bottom plate element. The sealing member may be installed automatically, using the manipulator. Alternatively, the respective elements are initially provided with the sealing member.

Further, the smart tank may comprise assembly reinforcement means for reinforcing the assembly of the top plate element, at least one of the sidewall elements and/or the bottom plate element of the smart tank. The assembly reinforcement means may include a threaded means, such as a threaded rod, a strap and/or a belt. For example, at least two of the elements may comprise a through opening for receiving a threaded rod. Those elements can then be assembled by using at least one threaded member, such as a nut, that threadedly engages with the threaded rod. Further, at least one strap or belt may be wrapped around the smart tank to reinforce the assembly of the elements. Further, the assembly reinforcement means may improve the tightness of the reservoir of the smart tank. Thus, leakage can be prevented, and the assembly of the smart tank can be facilitated. The assembly reinforcement means may be installed automatically, using the manipulator.

The smart tank may comprise multiple side-wall elements, wherein the top plate element, the sidewall elements and the bottom plate element are arranged to form the reservoir. This allows a small packing size of the disassembled smart tank (i.e. the smart tank assembly).

The smart tank may comprise multiple sidewall elements that can be assembled using the manipulator. All sidewall elements may be arranged in the same level of the smart tank. Alternatively, the side wall elements may be arranged in different levels of the smart tank. In case all sidewall elements are arranged in the same level of the smart tank, the stack of elements in the assembled smart tank is as follows: bottom plate element / side wall element / top plate element. In case the side wall elements are arranged in different levels of the smart tank, the stack of elements in the assembled smart tank may be as follows: bottom plate element /side wall element / ... / sidewall element / top plate element. Accordingly, in the stack at least one side wall element follows a sidewall element. This allows to form smart tanks having different volumes, using the same elements.

Any one of the top plate element, the at least one sidewall element and/or the bottom plate element may comprise at least one first channel portion and a at least one channel-connecting means being associated with a respective first channel portion. Additionally, a different one of the top plate element, the at least one sidewall element, a further side wall element and/or the bottom plate element may comprise at least one second channel portion and a at least one corresponding channel-connecting means being associated with a respective second channel portion. The channel-connecting means and the corresponding channel-connecting means may be configured to engage with each other, so as to form a fluidically sealed channel connection, between the first channel portion and the second channel portion, so as to form the at least one channel. Particularly, the element-interconnecting port and/or a corresponding element-interconnecting port (as described above) may include a channel-connecting means and/or a corresponding channel-connecting means.

The channel-connecting means may comprise a protruding shroud that at least partially surrounds the end of the associated first channel portion. The shroud may be concentrically arranged around a channel end of the associated channel portion. Further, the corresponding channel-connecting means may comprise a recess, that at least partially surrounds the end of the associated second channel portion. The recess may be concentrically arranged around the channel end of the associated second channel portion. The channel-connecting means and the corresponding channel-connecting means may be formed to provide a positive locking. For example, an element of the smart tank (top plate element, sidewall element and/or bottom plate element) may comprise a channel-connecting means. A further element of the smart tank (top plate element, sidewall element and/or bottom plate element) may comprise a corresponding channel-connecting means. This allows to couple the elements to form the reservoir of the smart tank and thereby to fluidically connect the respective channel portions of the elements to form a connected channel, when assembling the smart tank using the manipulator. Thus, a medium can be guided though the channel formed in at least two elements of the smart tank. Thereby assembling a smart tank is facilitated and less prone to assembly mistakes.

The element (top plate element, sidewall element and/or bottom plate element) may comprise multiple channel-connecting means and/or the corresponding channel-connecting means to provide a channel-connecting interface that allows to easily assemble the smart tank. The channel-connecting interface may be configured to allow assembly of different elements. For example, a sidewall element may be assembled with a top plate element or a further sidewall element, using the same channel-connecting interface.

The smart tank may further comprise at least one pumping means, wherein the pumping means may be separated from the reservoir and/or the at least one channel by a flexible membrane, so as to prevent direct contact between the at least one biochemical medium and the pumping means. The pumping means may be installed and assembled, using the manipulator.

The smart tank may further comprise at least one stirring means, wherein the stirring means may be drivable from the outside of the smart tank. For example, the stirring means may be driven by a magnetic actuator. Using a magnetic actuator allows to drive the stirring means from the outside of the smart tank and facilitates the sealing, as the magnetic actuator can be separated from the actual stirring means, e.g. by a continuous wall portion. The stirring means may be installed and assembled, using the manipulator.

The smart tank may further comprise at least one blending means, such as a fluid deflection plate, which may be integrally formed with either one of the sidewall elements, the top plate element and/or the bottom plate element. Further, the blending means may be a separate means that is provided in the reservoir of the smart tank. The blending means may be installed and assembled, using the manipulator.

The smart tank may further comprise at least one a cell-harvest-means. The cell-harvest means may include a filter and/or a filter cartridge that can be coupled or integrated in an element, particularly the bottom plate element of the smart tank. The cell-harvest-means may be installed and assembled, using the manipulator.

The smart tank may further comprise at least one cartridge for chromatography, so as to allow carrying out a preparative chromatography. The cartridge for chromatography may be installed and assembled, using the manipulator. Further, the smart tank may comprise at least one resin means. The resin means may be installed and assembled, using the manipulator The smart tank may further comprise at least one cross-flow cassette, so as to allow carrying out crossflow filtration or tangential flow filtration within the smart tank. The cross-flow cassette may be installed and assembled, using the manipulator.

The smart tank may further comprise at least one hollow-fibre means for cell harvesting, dia-filtration, ultra-filtration, and/or the like. The hollow-fibre means may be provided in a cartridge that can be integrated in the reservoir of the smart tank or that can be arranged outside of the reservoir of the smart tank. For example, the hollow-fibre means may be coupled to at least one of the elements of the smart tanks, using the manipulator.

The smart tank may be connectable to at least one sensor or a sensor module, comprising multiple sensors, wherein the at least one sensor and the sensors of the sensor module are chosen from the group of a pH sensor, a temperature sensor, a dissolved oxygen sensor, a biomass sensor, a foam sensor, a pressure sensor, a flow sensor, an O₂ sensor, an N₂ sensor, a CO₂ sensor, or the like. Further, the at least one sensor may be a spectroscopy means, such as RAMAN, NIR and/or UV spectroscopy means. Further, the sensor or sensor module may be connected to the smart tank, using the manipulator.

Further, when providing multiple assembled smart tanks, that shall be interconnected with each other, a height dimension of a first smart tank may be smaller than a height dimension of a second smart tank. For compensating the difference in height and for allowing a proper tank interconnection, at least one height compensation means may be provided. The height compensation means may be adapted to be coupled to the first smart tank, so that the top plate element of the first smart tank is installed at substantially the same height as the top plate element of the second smart tank, when the height compensation means is coupled to the first smart tank. The coupling of the height compensation means to the respective smart tank is preferably carried out automatically, using the manipulator.

The height compensation means allows that all top plate elements of the multiple smart tanks of a smart tank system can be arranged at substantially the same height. Thus, ports of the smart tanks, filters of the smart tanks and/or actuating means of valves of the smart tanks can be accessed and operated at substantially the same height. Thereby automated actuation and operation of the smart tank system and the process line is facilitated.

The height compensation means may comprise a platform for receiving the smart tank and a stand. The stand may be variable in height. Thus, a height compensation means may be used with different kinds of smart tanks. For example, the stand may comprise a telescope mechanism or a folding mechanism. A stand that is variable in height may be actuatable, e.g. by an electric or hydraulic drive, so as to provide an automated height variation. Alternatively, the manipulator may adjust the height of the height compensation means. Further, the smart tank and/or the height compensation means may comprise at least one wheel. Said at least one wheel facilitates moving the smart tank to form e.g. a smart tank system comprising multiple smart tanks. The wheel may be associated with a brake. Thus, undesired movement of the smart tank can be prevented.

Further, for interconnecting smart tanks of a smart tank system, a connection plate element may be provided. This connection plate element may be assembled to the smart tank(s) using the manipulator. Said connection plate element may include at least one channel and may be adapted to interconnect smart tanks that are not provided directly adjacent to each other. Using a connection plate element facilitates interconnecting spaced apart smart tanks. Those spaced apart smart tanks can e.g. sandwich one or more further smart tanks. Further, a connection plate element may be configured to multiplex or demultiplex a medium flow. For example, the connection plate element may contain a channel junction or a channel manifold. Thus, a medium received from a first smart tank can be supplied to multiple other smart tanks (multiplexing). Further, different mediums can be mixed within the connection plate element and can then be jointly supplied to a single smart tank (demultiplexing). Further, the connection plate element may be integrally formed with any one of the top-plate element, the at least one side wall element and/or the bottom plate element.

The system may further comprise at least one mobile delivery means, such as an automated guided vehicle. The mobile delivery means may be adapted to transport the at least one mobile storage means to the assembly room and/or to a sterilization station. The sterilization station may be configured to sterilize the mobile storage means by autoclaving or ETO, or the like. Preferably, the sterilization station allows for automated sterilization. Optionally, the at least one mobile delivery means is adapted to transport mobile storage means to the assembly room in a predefined order. Thus, the assembly order of multiple smart tanks can be controlled and a smart tank system, comprising multiple smart tanks and/or a bio-pharma process line can be assembled. The assembly room may be an assembly bag, comprising flexible wall elements, the assembly bag being optionally a sterile bag, that is further optionally a single-use bag.

Preferably the assembly bag is foldable, due to the flexible wall elements and can be delivered in a small packaging space. The wall elements comprise ceiling elements, bottom elements and/or sidewall elements. The elements may be partially or entirely flexible. For example, a wall element may comprise a rigid (foldable) support structure and flexible wall portions, being formed by a film, foil or the like.

The assembly bag may be initially sterilized or maybe sterilizable at the site of the biopharmaceutical goods manufacturer. A sterile assembly bag that can be connected to a sterilized mobile storage means allows assembling the at least one smart tank in a sterile environment. Thus, risk of contaminating the assembled smart tank is minimized. The risk can even further be minimized, if a single-use bag is used.

Further, by using a single-use bag, the bag can be discarded after use and expensive cleaning and testing of the assembly room can be avoided. This saves time and money. Further, a previous process line can be quickly replaced by a new process line, in case single-use bags are used.

The manipulator may be provided within the assembly room. Alternatively, only parts of the manipulator, such as an actuator, may be provided within the assembly room. For example, the manipulator may penetrate a flexible wall element of the assembly room, wherein the flexible wall element is sealed against the manipulator. Thus, parts of the manipulator, such as an actuator, can be provided within the assembly room, wherein other parts (such as a power unit, motors,...) are provided outside. This allows to provide an assembly room with smaller dimensions. Accordingly, clean room conditions and/or sterile environment can be maintained ore easily.

Further, the manipulator may be installed entirely outside the assembly room and may assemble the smart tank(s) within the assembly room while being separated by a flexible membrane. For example, the manipulator may access the assembly room for assembling the smart tank(s) via a glove, that is part of a flexible wall element of the assembly room. The glove may be connected to the assembly room via flexible foil portions, so that the manipulator is not hindered. Further, the glove may be coupled to the assembly room via a coupling such as a rotary coupling that allows to rotate the glove relative to the assembly room. Optionally, the manipulator may be at least partially covered by a manipulator bag. The manipulator bag may build a barrier between the manipulator and the assembly room. The manipulator bag may be integrally formed with the assembly bag.

The system may further comprise at least one clean room bag, wherein the clean room bag is configured to receive at least one assembled smart tank from the assembly room. Optionally, the clean room bag is configured to receive multiple interconnected smart tanks from the assembly bag, wherein the at least one clean room bag is adapted to sealingly couple to the assembly room. The clean room bag may server for storing and/or operating the assembled smart tank(s).

Sealingly coupling the clean room bag with the assembly room may be achieved using sealable openings, described above with respect to the mobile storage means and the assembly room.

Accoringly, the clean room bag may comprise at least one sealable opening. The sealable opening of the clean room bag may be coupled to a corresponding sealable opening of the assembly room so that both openings may be configured to open jointly, when the assembly room is sealingly coupled to the clean room bag. Thus, transferring assembled smart tanks from the assembly room to the clean room bag is facilitated.

The sealable opening of the clean room bag may be covered by at least one cover element. The corresponding sealable opening of the assembly room may be covered by at least one corresponding cover element. The at least one cover element and the at least one corresponding cover element are configured to couple with each other to open jointly.

For example, the sealable opening and the corresponding sealable opening may be provided as sliding doors. The at least one cover element/corresponding cover element maybe a door leaf of the respective sliding door. Coupling the cover element(s) with the corresponding cover element(s) can be achieved e.g. by magnetic means, positive locking means, or any other coupling or a locking means. Preferably, magnets (permanent of electric) are distributed at least on an edge portion of the cover element(s)/corresponding cover element(s). This allows coupling the cover element(s) with corresponding cover element(s) in a defined manner. Thus, the non-sterile outer faces of the cover element(s)/corresponding cover element(s) cover each other after coupling. Thus, the risk for contaminating the assembled smart tank(s) when being transferred through the sealable openings from the assembly room to the clean room bag can be further reduced.

The cover element(s)/corresponding cover element(s) are movable with respect to the clean room bag and/or the assembly room. The cover element(s)/corresponding cover element(s) and the clean room bag /assembly room may be sealed, e.g. by at least one brush seal, a lip seal and/or the like. Even more preferably, the cover element(s)/corresponding cover element(s) may be connected with the clean room bag, or the assembly room via a flexibel portion, such as a sealing foil portion. The flexibel portion may be connected to the cover element on a first side and to the clean room bag on a second side. Likewise, the flexibel portion may be connected to the corresponding cover element on a first side and to the assembly room on a second side. Thus, the sealable opening can be opened/closed without providing a gap between the cover element and the clean room bag and/or the corresponding cover element and the assembly room, respectively. Thus, the contamination risk can be further reduced.

The sealing frame described above may also be configured to frame the sealable opening of the clean room bag. Thus, an additional seal is provided that seals the opening from the environment, when being opened. Thus, the contamination risk can be further reduced. The sealing frame may be a separate frame, may be provided on the clean room bag and/or the assembly room.

Accoringly, a UV-light source may be provided that is associated with the sealable opening of the clean room bag. Thus, possible contaminants on the outer surface of the sealable opening can be removed/destroyed prior to and/or during opening the sealable openings.

The clean room bag may be a sterile bag and optionally a single-use bag. Preferably the clean room bag is foldable, due to flexible wall elements and can be delivered in a small packaging space. The wall elements comprise ceiling elements, bottom elements and/or sidewall elements. The elements may be partially or entirely flexible. For example, a wall element may comprise a rigid (foldable) support structure and flexible wall portions, being formed by a film, foil or the like.

The clean room bag may be initially sterilized or maybe sterilizable at the site of the bio-pharmaceutical goods manufacturer. A sterile clean room bag that can be connected to the assembly room allows transferring the assembled smart tank(s) from a first sterile or clean room environment to a second sterile environment. Thus, risk of contaminating the assembled smart tank is minimized. The risk can even further be minimized, if a single-use bag is used. A clean room bag can be installed in almost every environment, e.g. in a conventional factory hall. Thus, expensive and time-consuming planning and constructing of pharma-process line plants can be avoided. Thus, the biopharma process line can be set up very quickly. Further, as the clean room bag as such provides at least clean room conditions, no expensive conventional clean rooms have to be installed. Further, in case of a single-use clean room bag, expensive and time-consuming cleaning and testing can be avoided.

The assembly bag and/or the clean room bag may be held by a support structure. The support structure may be a rigid support structure, such as a frame. Alternatively, the support structure may be inflatable. It is also possible, that the assembly bag and/or the clean room bag as such can be inflated. Thus, a mobile and self-standing assembly system can be provided.

For operating the smart tank or a system comprising multiple smart tanks, an adaptor plate element may be provided. The adaptor plate element is adapted to be arranged outside the clean room bag and to be coupled to an assembled smart tank, to cover a port of the smart tank and/or a filter at least partially, so as to give access to the smart tank from the outside of the clean room bag.

The adaptor plate element and the top plate element may sandwich a barrier element, wherein the barrier element is part of a clean room bag. In this case, the adaptor plate element may be arranged outside the clean room and the top plate element (as well as the at least one sidewall element and the bottom plate element) are arranged inside the clean room.

Further, the adaptor plate element may provide access to at least one port of the smart tank, to at least one filter of the smart tank and/or to at least one actuating means of a valve of the smart tank. In case the barrier element is sandwiched between the adaptor plate element and the top plate element, the top plate element as well as the at least one sidewall element and the bottom plate element of the smart tank can be maintained in the clean room wherein access to the smart tank from the outside of the clean room is possible via the access provided by the adaptor plate element. Thus, a biochemical medium and/or an operating medium can be supplied to and/or removed from the smart tank from the outside of the clean room. Further, the at least one valve can be actuated and controlled from the outside of the clean room bag.

The clean room bag may comprise at least one rail for guiding the at least one received assembled smart tank within the clean room bag. Thus, positioning the assembled smart tank within the clean room bag is facilitated. For example, the manipulator may set an assembled smart tank on the rail. After having assembled a further smart tank, this further smart tank is interconnected with the previously assembled smart tank and also set on the rail. Thereby, the previously assembled smart tank can be guided further into the clean room bag. Repeating this, an entire process line can be assembled and guided into the clean room bag.

The mobile storage means may comprise a tracking means, that may allow a geographical and/or local tracking of the mobile storage means. The tracking means may comprise a satellite-based tracking means, such as a receiver for GPS, GLONASS, Galileo and/or the like. Additionally, or alternatively the tracking means may comprise a NFC tag, a RFID tag, a barcode, a QR-code, a radio transmitter and/or the like. Thus, the mobile storage means can be tracked within a manufacturing site as well as during transport. This improves the delivery logistics. For example, the stock of components/mobile storage means may be tracked at a manufacturer. This allows automatically ordering further components or respective mobile storage means. Particularly, the number and content of mobile storage means that are presently in transit may be known, as well as the estimated delivery time. Thus, new orders can be placed in time, so as to guarantee that the manufacturer will not run out of stock. This improves manufacturing and logistics for manufacturing biopharmaceutical goods in the biopharma process line.

The mobile storage means may be adapted to allow sterilization of smart tank components being stored within the mobile storage means. As described above, sterilization may be carried out using ETO gas, autoclaving, gamma radiation or any other known sterilization technique.

Particularly, the mobile storage means may be adapted to maintain the sterilized state of smart tank components being stored within the mobile storage means. This can be achieved by providing a sealed mobile storage means that prevents entry of contaminants. Thus, the sterilized mobile storage means may be transported and stored for subsequent use.

The mobile storage means may include a sterile indicator. The sterile indicator may be adapted to indicate whether the components stored in the mobile storage means were sterilized within the mobile storage means and/or whether the sterilization has been expired (e.g. due to lapse of time, or entry of contaminants). E.g., the sterile indicator may change its color when being sterilized. Thus, it can be tracked whether the mobile storage means was sterilized. Further, the sterile indicator may indicate whether contaminants have entered the mobile storage means. Entry of contaminants can e.g. be detected, after the mobile storage means was evacuated after sterilization or filled with a sterile inert gas. Entry of oxygen (or any other gas) to the mobile storage means can be detected and indicates that the mobile storage means is no longer sterile.

Further, the mobile storage means may comprise a port for receiving sterilized air. The port may be covered by a filter, preferably a sterile filter. Thus, conventional pressurized air can be provided that enters the mobile storage means in a sterile fashion. Thus, a sterile leakage testing of the mobile storage means can be performed. For this, the mobile storage means is pressurized and the change of pressure over time is measured. In case the change of pressure exceeds a predefined threshold, the mobile storage means was not properly sealed and cannot be considered as being sterile.

The mobile storage means may include at least one locking element, that is adapted to lock the mobile storage means during sterilization and/or transportation. Thus opening the mobile storage means unintentionally can be prevented and the sterilized state can be maintained.

Further, the mobile storage means may include a transport bag, that is preferably a sterile bag. The transport bag may house the smart tank components being stored within the mobile storage means. This facilitates transferring the components from the mobile storage means to the assembly room, as the sterile components can be transferred within the transport bag to the assembly room. The transport bag can then be opened in the assembly room. Optionally the transport bag is an assembly bag. Thus, the components need not to be transferred to the assembly room but can be assembled directly in the transport/assembly bag. Thus, contamination risk of the components and/or the assembled smart tank can be reduced. Still further, the transport bag may remain in the mobile storage means and the components are removed from the transport bag, while the transport bag is still held in the mobile storage means. In this case, the transport bag may comprise a sealable opening that is adapted to open jointly with the sealable opening of the mobile storage means.

Further, the sealable opening of the transport bag may be adapted to be coupled directly with the corresponding sealable opening of the assembly room, so that the sealable opening of the transport bag and the corresponding sealable opening of the assembly room are configured to open jointly, when the transport bag is sealingly coupled to the mobile storage means.

Even more preferred, the transport bag comprises a rupture line that is associated with the sealable opening of the mobile storage means. Thus, when the sealable opening of the mobile storage means is opened, the transport bag is teared open along the rupture line in a defined manner.

The manipulator may be further adapted to disassemble smart tanks and optionally to remove assembled smart tanks from the clean room bag. This allows an automated disassembly or change of a bio-pharma process line and/or of a system of multiple smart tanks.

The system may further comprise at least one recycling means, adapted for receiving disassembled components of a smart tank and preferably for shredding said components. The recycling means may be a mobile storage means that is adapted to receive disassembled components of a smart tank from the assembly room, that may serve as disassembly room. The disassembly of the smart tanks may be carried out by the assembly manipulator. The recycling means may be adapted to sealingly couple to the assembly room. Optionally, the recycling means comprises at least one sealable opening that is configured for receiving disassembled components from the assembly room, wherein the sealable opening of the recycling means and the corresponding sealable opening of the assembly room are configured to open jointly, when the assembly room is sealingly coupled to the recycling means. Thus, disassembled components can be removed from the system without contaminating the sterile and/or clean room environment.

Further optionally, the sealable opening of the recycling means may be covered by at least one cover element, wherein the at least one cover element of the recycling means and the at least one corresponding cover element of the assembly room are configured to couple with each other to open jointly. Thus, the same advantages can be achieved as described above with respect to the sealable opening of the mobile storage means and/or the sealable opening of the clean room bag. Particularly, the sealable opening of the recycling means may be associated with a sealing frame and/or a UV-light source.

The object is at least partially achieved by a method for assembling a modular smart tank for a bio-pharma process, the method comprising the following steps: providing at least one mobile storage means that stores components of at least one smart tank; providing an assembly room that is adapted to provide clean room conditions for assembling the modular smart tank, wherein the assembly room may be an assembly bag; providing a manipulator, and automatically assembling the smart tank within the assembly room from the components stored in the mobile storage means, using the manipulator. The method allows to achieve the advantages described above.

The method may further comprise providing a clean room bag and coupling said clean room bag with the assembly room. This allows to transfer assembled smart tanks from the assembly room to the clean room bag. Further, the method may comprise interconnecting an assembled smart tank with a further assembled smart tank and moving the interconnected smart tanks from the assembly room to the clean room bag. Thus, the interconnected smart tanks may be operated or stored within the clean room bag.

The object is at least partially achieved by a mobile storage means for being used in a system as described above. The mobile storage means is adapted for storing components of at least one smart tank. Further, the mobile storage means may be adapted to sealingly couple to the assembly room of the system. The mobile storage means includes at least one sealable opening that is configured for allowing removal of components of the at least one smart tank from the mobile storage means, wherein the sealable opening of the mobile storage means is configured to open jointly with a corresponding sealable opening of an assembly room of the system, when the assembly room is sealingly coupled to the mobile storage means. Optionally, the sealable opening of the mobile storage means may be covered by at least one cover element, and the at least one cover element of the sealable opening of the mobile storage means may be configured to couple with a corresponding cover element of the corresponding sealable opening of the assembly room so that the sealable opening of the mobile storage means and the corresponding sealable opening of the assembly room open jointly. The mobile storage means may have any configuration described above.

Further, the object is at least partially achieved by an assembly bag, for being used in a system as described above. The assembly bag is adapted to provide clean room conditions for assembling at least one smart tank from components stored in a mobile storage means. The assembly bag may comprise at least one corresponding sealable opening that is configured for receiving components from a mobile storage means, wherein the corresponding sealable opening of the assembly bag is configured to open jointly with a sealable opening of a mobile storage means, when the assembly bag is sealingly coupled to the mobile storage means. Further optionally, the corresponding sealable opening of the assembly bag may be covered by at least one corresponding cover element and the at least one cover element of the corresponding sealable opening of the assembly bag may be configured to couple with a cover element of the sealable opening of the mobile storage means so that the sealable opening of the mobile storage means and the corresponding sealable opening of the assembly room open jointly. The assembly bag may have any configuration described above.

Further, the assembly bag and/or the clean room bag may comprise a multi-ply outer shell, wherein a first ply of the shell is the outermost ply, and wherein a second ply of the shell is the innermost ply. A space surrounded by the second ply of the shell is pressurized with a pressure being lower than a pressure provided between the first and the second ply of the shell. A colored gas may be provided between the first and the second ply of the shell. Thus, when the second ply of the shell would leak, colored gas would enter the space surrounded by the second ply. This can be detected and hints at entry of contaminants in the sterile and/or clean room environment.

The assembly bag may be initially stored in a frame assembly. The frame assembly may comprise air-permeable wall elements, wherein the assembly bag may comprise an air inlet port that serves for pressurizing the stored assembly bag for leakage testing. Thus, the assembly bag can be pressurized while being stored in the frame assembly and the change of pressure over time can be measured. As the frame assembly comprises air-permeable wall elements, air that leaves the stored assembly bag can also leave the frame assembly and thus, leakages can be detected. The air-permeable wall elements may be provided in form of wall elements with through holes, such as punctured sheets and/or in form of wall elements that are covered with an air-permeable means, such as an open-pored foam, a fabric, a non-woven, and/or the like. Particularly, leakages can be detected by comparing the measured change of pressure over time with a predefined threshold. In case the change of pressure over time exceeds the threshold, there is leakage and the assembly bag cannot be used.

### Brief Description of the Figures

In the following, the accompanying figures, that schematically show embodiments of the invention are described. Here,
- Fig. 1: schematically shows a smart tank assembly in a disassembled state;
- Fig. 2: schematically shows a smart tank in an assembled state;
- Fig. 3A: schematically shows a smart tank system;
- Fig. 3B: schematically shows a further smart tank system;
- Fig. 4: schematically shows smart tanks, having different volumes;
- Fig. 5: schematically shows an assembly system;
- Fig. 6: schematically shows an assembly system, comprising a clean room bag;
- Fig. 7A: schematically shows a sealable opening being coupled to a corresponding sealable opening in an open state;
- Fig. 7B: schematically shows a sealable opening being coupled to a corresponding sealable opening in a closed state;
- Fig. 7C: schematically shows a sealing frame;
- Fig. 8A: schematically shows a system, comprising a transport bag;
- Fig. 8B: schematically shows the system of Fig. 8A;
- Fig. 9: schematically shows an assembly bag in an initial state, and
- Fig. 10: schematically shows a flow diagram of a method.

### Detailed description of the Figures

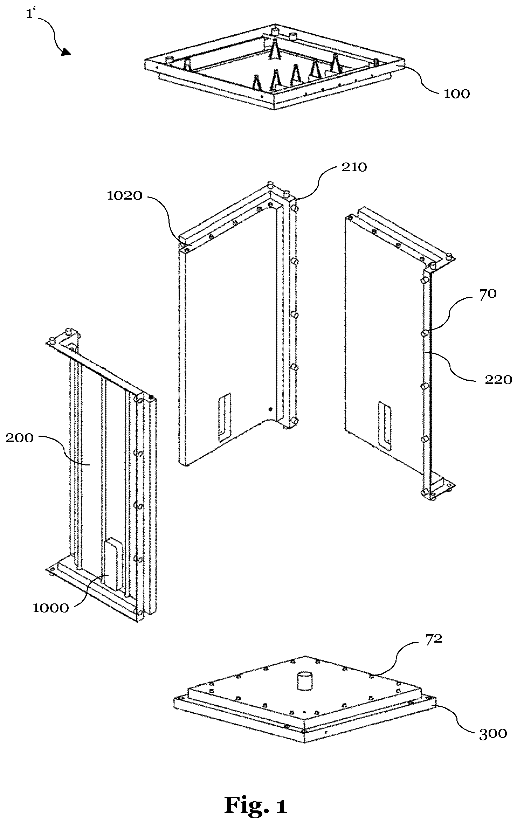

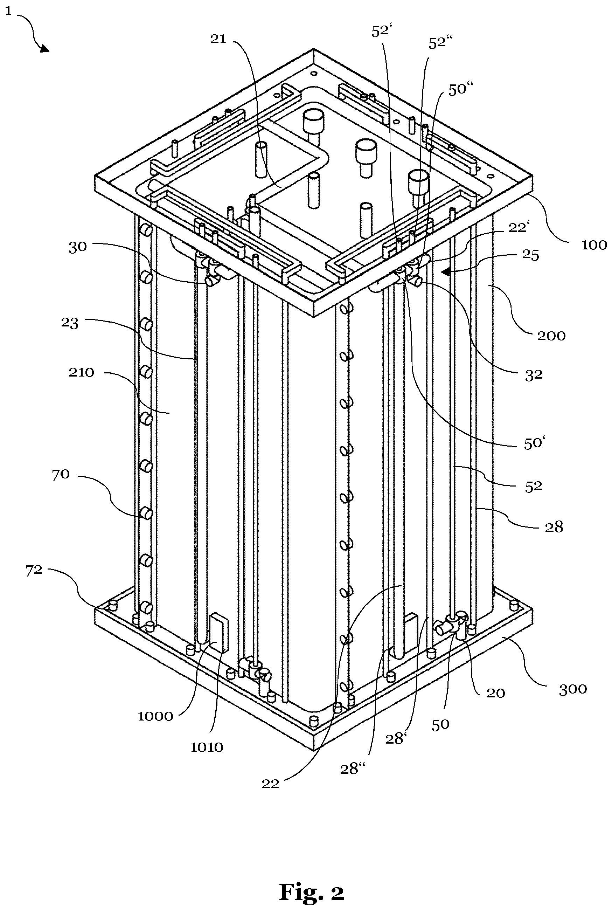

In particular, Fig. 1 schematically shows a smart tank assembly 1' in a disassembled state. The smart tank assembly 1' is adapted to be assembled to a smart tank 1 (as e.g. shown in Fig. 2) using the system for assembling a smart tank. The smart tank assembly 1' comprises a top plate element 100, at least one sidewall element 200, 210, 220, and a bottom plate element 300. As depicted, the smart tank assembly 1' of Fig. 1 comprises three sidewall elements 200, 210, 220.

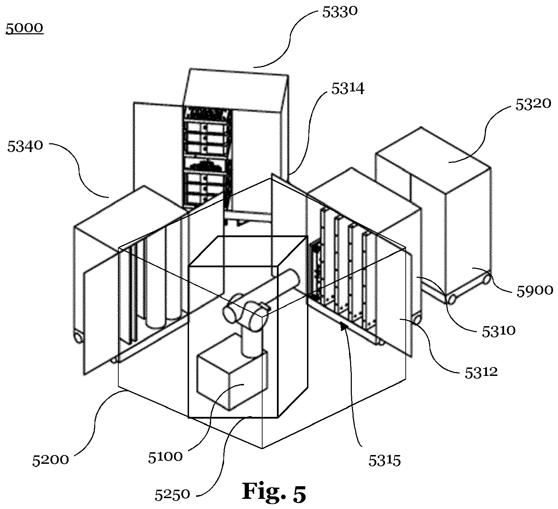

The top plate element 100, the sidewall elements 200, 210, 220 and the bottom plate element 300 can be assembled, using a manipulator of the system, to form a reservoir 500 for receiving at least one biochemical and/or operating medium. The manipulator of the system (cf. e.g. Fig. 5) may be adapted to assemble any other component of the smart tank, such as a valve, a filter, a sensor and/or the like.

At least one of the top plate element 100, the sidewall elements 200, 210, 220 and the bottom plate element 300 comprises at least one channel, for guiding the at least one biochemical medium and/or an operating medium. The channel may be associated with a valve that allows to open/close the channel. The valve may also be a flow control valve, that allows to control the flow through the channel. The channel may further comprise a port, such as an inlet and/or outlet port.

The smart tank assembly 1' may comprise at least one sealing member 1020 for providing a sealed connection between the elements (top plate element 100, sidewall elements 200, 210, 220 and bottom plate element 300). The sealing member 1020 may be arranged circumferentially at each sidewall element 200, 210, 220, top plate element 100 and/or bottom plate element 300. When the elements are assembled to form the reservoir 500, the sealing member 1020 may be compressed, so as to provide a retaining force that acts on assembly-connecting means 70 and corresponding assembly-connecting means 72. Thereby, a self-retaining engagement maybe provided.

Each of the elements (top plate element 100, sidewall elements 200, 210, 220 and bottom plate element 300) may be adapted to be provided with a sensor 1010 and/or a sensor module 1000. A sensor module 1000 may comprise multiple sensors, such as at least one of a pH sensor, a temperature sensor, a dissolved oxygen sensor, a biomass sensor, a foam sensor, a pressure sensor, a flow sensor, an O2 sensor, a N2 sensor, a CO2 sensor, and spectroscopy means, such as RAMAN, NIR and/or UV spectroscopy means. The sensor module may be connectable to the respective top plate element 100, sidewall element 200, 210, 220 and/or bottom plate element 300. The sensor module 1000 maybe provided with a power source such as a rechargeable battery, that allows to operate the sensor module 1000 autonomously. Further, the sensor module 1000 may comprise a data interface, particularly a wireless data interface for transferring the measured sensor data to a respective control or storing unit.

Fig. 2 shows a smart tank 1 in an assembled state. This smart tank can e.g. be achieved by an assembly system (cf. Fig. 5) or a method for assembling a smart tank (cf. Fig. 12). This smart tank may server for storing and/or transporting a biochemical medium. The shown smart tank comprises a top plate element 100, multiple sidewall elements 200, 210, and a bottom plate element 300. The top plate element, the sidewall elements and the bottom plate element are arranged to form a reservoir for receiving at least one biochemical medium.

The smart tank 1 comprises further channels 20, 21, 22, 23 for guiding the at least one biochemical medium and/or an operating medium. The channels extend within at least one of the top plate element 100, the sidewall elements 200, 210 and/or the bottom plate element 300.

For example channel 21 extends in the sidewall element 200 and the top plate element 100. Other channels may be provided that extend in the at least one sidewall element and at least one of the top plate element and the bottom plate element. Each of the channels may be at least one of the following channel types: An inlet channel, for guiding a biochemical medium and/or an operating medium to the reservoir of the smart tank. An outlet channel, for removing a biochemical medium and/or an operating medium from the reservoir of the smart tank. A retentate channel, for transferring a retentate back into the smart tank or out of the smart tank. A bypass-channel, for guiding a biochemical medium and/or an operating medium, wherein the bypass-channel is not connected to the reservoir of the smart tank. Alternatively, the bypass-channel can be adapted to be fluidically separated from the reservoir of the smart tank, e.g. by means of a valve. A heating or cooling channel for guiding a tempered heating or cooling medium. A sampling channel, for taking a sample of biochemical medium and/or of operating medium from the reservoir of the smart tank. Particularly, the smart tank may comprise multiple channels of different channel-types and/or the same channel type.

In the smart tank shown in Fig. 2, channel 20 serves as outlet channel, particularly for removing waste. Channel 22 can be either an inlet or an outlet channel. This channel 22 enters the reservoir at a bottom side of the smart tank (i.e. near the bottom plate element). Channel 22' can also be either an inlet or an outlet channel. This channel 22 enters the reservoir at a top side of the smart tank (i.e. near the top plate element). Channel 21 is a bypass-channel, that allows guiding a biochemical medium and/or an operating medium, via the smart tank, without entering the reservoir of the smart tank. Channels 28, 28' and 28" are not connected to the reservoir and may serve as cooling and/or heating channels.

Channels 21, 22 and 22' meet each other at a channel junction 25. This channel junction is provided with at least one (in the embodiment shown with two) valves 50', 50". These valves 50', 50" are actuatable from the outside of the smart tank, by means of an actuating means 52', 52". The actuating means 52', 52" are provided in form of actuation rods. Channel 20 is associated with a respective valve 50 which can be actuated from the outside of the smart tank, by means of actuating means 52.

Further, the smart tank 1 comprises ports 30, 32. The ports are each associated with respective channel(s). The ports may be chosen from a group of port-types, comprising the following port-types: a fluid inlet port, a gas inlet port, a fluid outlet port, a gas outlet port, a cell blead port, a medium supply port, a medium remove port, an element-interconnecting port, and a tank-interconnecting port.

For example, port 32 is associated via valves 52', 52" with channels 21, 22 and 22'. Accordingly, this port 32 may serve as fluid inlet port, gas inlet port, fluid outlet port, gas outlet port, medium supply port, medium remove port, or tank-interconnecting port. Chanel 20, which serves as outlet channel is associated with an outlet port (not shown) on a bottom side of the bottom plate element 300.

Particularly, all sides of the smart tank (or at least some of the sides) may provide the same port interface. I.e. ports are arranged at the same position. Thus, a smart tank can be easily interconnected to a further smart tank. Further, all side wall element of a smart tank may be identically structured. Thus, the number of different elements required for setting up a smart tank is reduced.

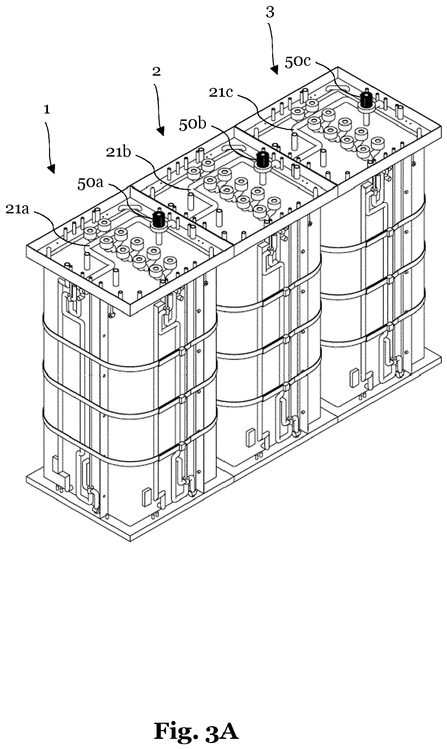

Fig.3A schematically shows a smart tank system that comprises multiple smart tanks 1, 2, 3. The smart tanks are directly interconnected with each other. The interconnection of the smart tank may be performed, using the manipulator of the system for assembling a smart tank (cf. e.g. Fig. 5). For example, the manipulator may assemble a first smart tank and guide the same in a clean room bag (not shown). After having assembled the first smart tank, a further, second smart tank is assembled. The second smart tank is then interconnected with the previously assembled first smart tank. The first and second smart tank can then be guided further into the clean room bag. Repeating this, a entire process line can be assembled and guided into the clean room bag bay the manipulator.

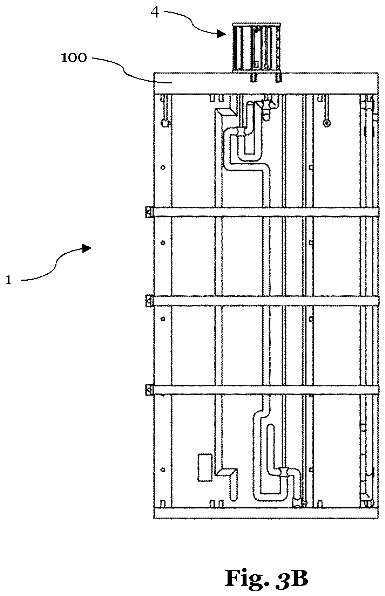

At least one of the one or more channels 21a of the first smart tank 1 is fluidically connected to a respective channel 21b of the second smart tank 2 which can be connected to a respective channel 21c of the third smart tank. Depending on the position of the valves 50a, 50b and/or 50c. Medium can be e.g. transferred from the first smart tank to the second smart tank or the third smart tank. When transferring medium to the third smart tank medium can either be guided through the reservoir of the second smart tank or medium can bypass the reservoir of the second smart tank. Fig. 3B schematically shows a further smart tank system. The smart tank system comprises two smart tanks, a larger smart tank 1 and a smaller small tank 4. The smaller smart tank 4 is provided on top of the larger smart tank 1. The smaller smart tank 4 can be provided on top of the larger smart tank 1 by using the manipulator (not shown). The manipulator may comprise a gripping device that is adapted to grip the smaller smart tank and to put it on top of the larger smart tank 1. Both smart tanks may be fluidically connected, via a channel or port (not shown). The channel or port may comprise a sterile filter, allowing to transfer medium from the smaller smart tank to the larger smart tank without contaminating the larger smart tank. The fluid connection may be controlled by a valve. The smaller smart tank may serve to provide an operating medium and/or a biochemical medium to the larger smart tank. As the smaller smart tank 4 is installed on top of the larger smart tank 1, it is possible to transfer medium from the smaller smart tank to the larger smart tank by using gravitation. Alternatively, medium can be transferred by providing a positive pressure to the smaller smart tank and/or by providing a negative pressure to the larger smart tank.

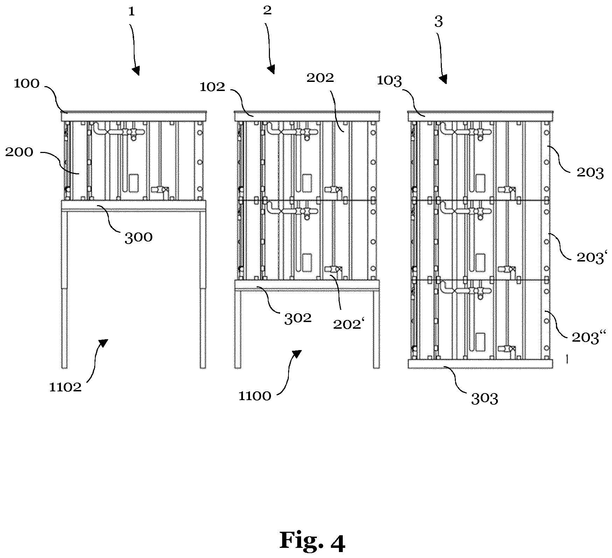

Fig.4 schematically shows smart tanks 1, 2, 3, having different volumes. To provide different volumes, it is possible to provide different kinds of sidewall elements. Thus, different volumes can be provided, using the same top plate element and bottom plate elements. Further, different volumes can be provided by stacking multiple sidewall elements 202, 202'; 203, 203', 203". As shown in Fig. 4, smart tank 1 comprises sidewall elements 200 that are all arranged in the same level of the smart tank 1. This smart tank has the following stack of elements: bottom plate element 300 / side wall element 200 / top plate element 100.

Smart tanks 2 and 3 comprises multiple sidewall elements 202, 202'; 203, 203', 203", wherein the groups of side wall elements are arranged in different levels of the smart tank. Smart tank 2 has the following element stack: bottom plate element 302 /side wall element 202' / sidewall element 202 / top plate element 102 and smart tank 3 has the following element stack: bottom plate element 303 / side wall element 203" / side wall element 203' / sidewall element 203 / top plate element 103.

Channel portions extending in the respective sidewall elements are interconnected with corresponding channel portions in the neighboring element (sidewall element, bottom plate element or top plate element). Likewise, actuating means for actuating a valve and/or for driving a stirring means and/or the like that are provided in the sidewall element(s) may be coupled to corresponding actuating means of a neighboring element. Thus, a valve or the like provided e.g. in sidewall element 202' or 203" can be actuated from the top side of the smart tank.

As shown, the height dimension of the first smart tank 1 is smaller than a height dimension of the second smart tank 2 and the third smart tank 3. To provide the top plate elements 100, 102 and 103 on substantially the same height, a height compensation means 1100, 1102 can be provided, preferably by the manipulator. Said height compensation means 1100, 1102 is adapted to be coupled to the smart tank 1, 2 and allows to install the top plate element 100 of the first smart tank 1, the top plate element 102 of the second smart tank 2 and in substantially the same height as the top plate element 103 of the third smart tank 3. Thus, interconnection of the smart tanks is facilitated.

Fig. 5 schematically shows an assembly system 5000 for assembling a modular smart tank for a bio-pharma process under clean room conditions. The system 5000 comprises at least one mobile storage means 5310, 5320, 5330, 5340 for storing components of at least one smart tank and an assembly room (5200). The assembly room 5200 is adapted to provide clean room conditions is configured to allow assembly of at least one smart tank within the assembly room from the components stored in the mobile storage means 5310, 5320, 5330, 5340. The system also comprises a manipulator 5100 that is adapted to automatically assemble the smart tank within the assembly room 5200. The manipulator 5100 may be provided with in the assembly room to assemble the smart tank(s) within the assembly room. Further, the manipulator 5100 may be separated from the clean room environment by a flexible membrane 5250 (e.g. a glove). Further, the assembly room may be adapted to sealingly couple to at least one mobile storage means 5310, 5320, 5330, 5340, preferably by sealable opening 5315. The sealable opening may be covered by cover elements 5312, 5314.

Further the system may comprise at least one mobile delivery means 5900, such as an automated guided vehicle. The mobile delivery means 5900 may be adapted to transport the at least one mobile storage means 5310, 5320, 5330, 5340 to the assembly room, preferably in a predefined order.

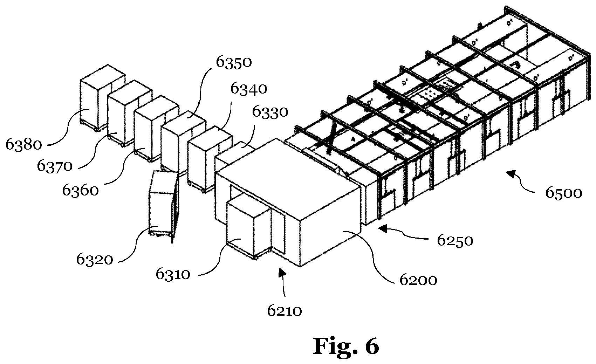

Fig. 6 schematically shows an assembly system 6000. The manipulator not shown, as it is provided inside the assembly room 6200, which may be an assembly bag. The assembly room 6200 provides clean room conditions for assembling smart tanks, from components being stored in mobile storage means 6310, 6320, 6330, 6340, 6350, 6360, 6370, 6380. The assembly room 6200 comprises at least three sealable openings 6210, 6250 for sealingly couple to at least one mobile storage means 6310 and 6250 and to a clean room bag 6500. The clean room bag serves for receiving assembled smart tanks from the assembly room 6200.

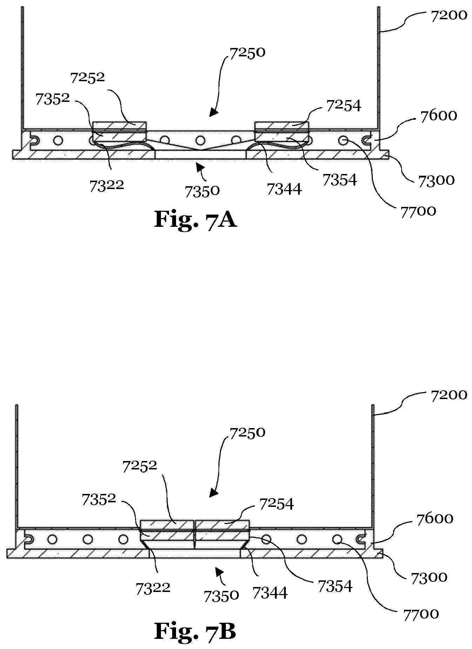

Fig. 7A and 7B schematically shows a sealable opening being coupled to a corresponding sealable opening. Fig. 7A shows an open state, wherein Fig. 7B shows a closed state. The sealable opening 7350 may be part of a mobile storage means 7300, of a clean room bag or of a recycling means. The corresponding sealable opening is part of an assembly room 7200, preferably of an assembly bag. In the following the opening/closing is described with respect to an assembly room being sealingly coupled to a mobile storage means. The described principle can be likewise transferred to the sealable opening of a clean room bag and/or of a recycling means.

The sealable opening 7350 is configured for allowing removal of components of the at least one smart tank from the mobile storage means 7300. The assembly room 7200 comprises at least one corresponding sealable opening 7250 that is configured for receiving components from a mobile storage means 7300. The sealable opening 7350 of the mobile storage means 7300 and the corresponding sealable opening 7250 of the assembly room 7200 are configured to open jointly, when the assembly room 7200 is sealingly coupled to the mobile storage means 7300.

Further, the sealable opening 7350 of the mobile storage means 7300 may be covered by at least one cover element 7352, 7354, such as a door leaf of a sliding door. The corresponding sealable opening 7250 of the assembly room 7200 may be covered by at least one corresponding cover element 7252, 7254. The at least one cover element 7352, 7354 and the at least one corresponding cover element 7252, 7254 are configured to couple with each other to open jointly. The coupling can e.g. be achieved magnetically or by positive locking.

Further, a sealing frame 7600 maybe provided. Here, sealing frame 7600 is integrally formed with the mobile storage means 7300. The sealing frame 7600 is configured to frame the sealable opening 7350 of the mobile storage means 7300 and the corresponding sealable opening 7250 of the assembly room 7200. When the openings 7350 and 7250 are closed, the space within the sealing frame 7600 maybe evacuated. Further the space may be sterilized using UV-light source 7700. The UV-light source 7700 is associated with the sealable opening 7350 of the mobile storage means 7300. The cover element(s) 7352, 7354 may be connected with the mobile storage means 7300 via a flexibel portion 7322, 7344, such as a sealing foil portion. The flexibel portion 7322, 7344 maybe connected to the cover element 7352, 7354 on a first side and to the mobile storage means 7300 on a second side. Likewise, a flexibel portion may be connected to the corresponding cover element of on a first side and to the assembly room on a second side. Thus, the sealable opening 7350 can be opened/closed without providing a gap between the cover element 7352, 7354 and the mobile storage means 7300 and/or the corresponding cover element and the assembly room, respectively.

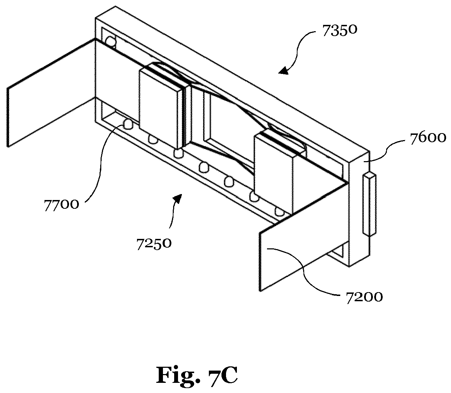

Fig. 7C schematically shows a sealing frame 7600'. The sealing frame 7600' is a separate frame and is configured to frame the sealable opening 7350 of the mobile storage means 7300 and/or the corresponding sealable opening 7250 of the assembly room 7200. The sealing frame 7600' may comprise a UV-light source 7700. The UV-light source may be adapted to destroy contaminants on the outer surface of the sealable openings prior to and/or during opening the sealable openings. Thus, the framed space may be sterilized. The sealing frame 7600' may further comprise a port that allows evacuating a space between the sealing frame and the closed sealable openings of the mobile storage means and/or the assembly room prior to opening the sealable openings. Thus, the risk of contamination can be further reduced. The sealing frame may frame the sealable opening 7350 of the mobile storage means 7300 and/or the corresponding sealable opening 7250 of the assembly room 7200 sealingly, after the at least one cover element 7352, 7354 and the at least one corresponding cover element 7252, 7254 are coupled.

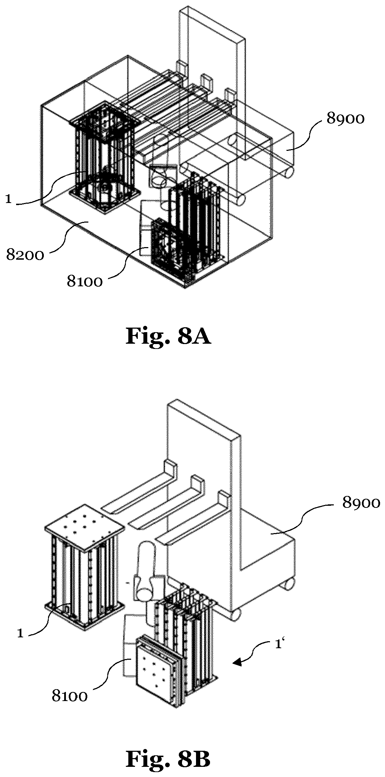

Further, the sealing frame may comprise a flexible frame portion. This allows to sealingly frame the sealable opening 7350 of the mobile storage means 7300 and/or the corresponding sealable opening 7250 of the assembly room 7200 prior to coupling the at least one cover element 7352, 7354 and the at least one corresponding cover element 7252, 7254. Accordingly, the sealing frame 7600' maybe able to UV irradiate outer surfaces of the at least one cover element 7352, 7354 and the at least one corresponding cover element 7252, 7254 and/or to remove particles and contaminants by evacuating, prior to coupling the coupling the at least one cover element 7352, 7354 and the at least one corresponding cover element 7252, 7254. Likewise, the integrally formed frame that is described with respect to Figs. 7A and 7B may comprise a respective flexible frame portion. Fig. 8A and Fig. 8B show an assembly system 8000 for assembling a modular smart tank for a bio-pharma process under clean room conditions. The system 8000 comprises at least one mobile storage means (not shown) for storing components of at least one smart tank and an assembly bag 8200. The assembly bag 8200 is adapted to provide clean room conditions and is configured to allow assembly of at least one smart tank within the assembly bag from the components stored in the mobile storage means. The mobile storage means includes a transport bag, that may be a sterile bag. The transport bag houses the smart tank components being stored within the mobile storage means, wherein in the system shown in Figs. 8A and 8B, the transport bag is the assembly bag 8200. The transport bag provides a secondary packaging for the components stored in the mobile storage means. Alternatively, the transport bag may be the mobile storage means.

The system also comprises a manipulator 8100 that is adapted to automatically assemble the smart tank 1 within the assembly bag. The manipulator 8100 is provided outside the assembly bag 8200, wherein the manipulator 8100 is at least partially covered by a manipulator bag that is integrally formed with the assembly bag 8200. For assembling the smart tank 1, the transport bag is removed from a mobile storage means and put over the manipulator 8100, using a mobile delivery means 8900. The transport bag includes components (smart tank assembly 1') for at least one smart tank that can be assembled using the manipulator 8100. During assembly, the mobile delivery means 8900 may hold the transport/assembly bag, as shown in Fig. 8A

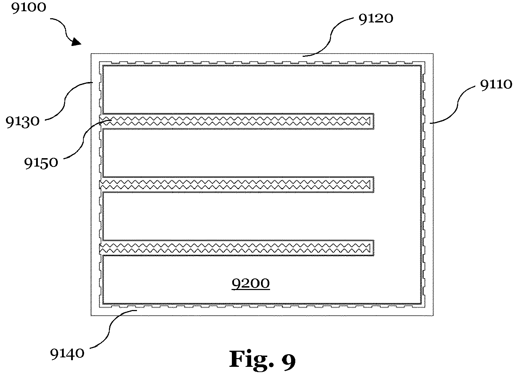

Fig. 9 shows an assembly bag 9200 in an initial sate, being stored in a frame assembly 9000. The frame assembly 9100 comprise air-permeable wall elements 9110, 9120, 9130, 9140, 9150. The air-permeable wall elements may be provided in form of wall elements with through holes, such as punctured sheets and/or in form of wall elements that are covered with an air-permeable means, such as an open-pored foam, a fabric, a non-woven, and/or the like. The assembly bag comprises an air inlet port (not shown) that serves for pressurizing the stored assembly bag 9200 for leakage testing. The air inlet port maybe covered with at filter, such as a sterile filter, that allows pressurizing the assembly bag in a sterile fashion. Thus, the assembly bag can be pressurized while being stored in the frame assembly 9000 and the change of pressure over time can be measured. Particularly, leakages can be detected by comparing the measured change of pressure over time with a predefined threshold. In case the change of pressure over time exceeds the threshold, there is leakage and the assembly bag cannot be used. As the frame assembly comprises air-permeable wall elements 9110, 9120, 9130, 9140, 9150, air that leaves the stored assembly bag 9200 can also leave the frame assembly 9100 and thus, leakages can be detected.

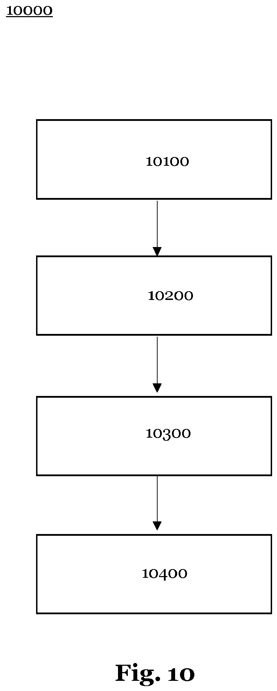

Fig. 10 shows a flow diagram of a method 10000 for assembling a modular smart tank for a bio-pharma process. The method 10000 comprises the following steps: Providing 10100 at least one mobile storage means that stores components of at least one smart tank. Providing 10200 an assembly room that is adapted to provide clean room conditions for assembling the modular smart tank, wherein the assembly room may be an assembly bag. Providing 10300 a manipulator. Automatically assembling 10400 the smart tank within the assembly room from the components stored in the mobile storage means, using the manipulator.

### Further Embodiments:

Embodiment 1. An assembly system 5000, 6000, 8000 for assembling a modular smart tank 1 for a bio-pharma process under clean room conditions, the system comprising:
   at least one mobile storage means 5300, 6300 for storing components of at least one smart tank 1,
   an assembly room 6200, 8200 , wherein the assembly room is adapted to provide clean room conditions and wherein the assembly room is configured to allow assembly of at least one smart tank within the assembly room from the components stored in the mobile storage means, and
   a manipulator 5100, 8100, being adapted to automatically assemble the smart tank within the assembly room.
Embodiment 2. The system 5000, 6000, 8000 of embodiment 1, wherein the assembly room is adapted to sealingly couple to at least one mobile storage means.
Embodiment 3. The system 5000, 6000, 8000 of any preceding embodiment, wherein the mobile storage means 7300 includes a sealable opening 7350 that is configured for allowing removal of components of the at least one smart tank from the mobile storage means, and wherein
   the assembly room 7200 comprises at least one corresponding sealable opening 7250 that is configured for receiving components from a mobile storage means, wherein
   the sealable opening of the mobile storage means and the corresponding sealable opening of the assembly room are configured to open jointly, when the assembly room is sealingly coupled to the mobile storage means.
Embodiment 4. The system 5000, 6000, 8000 of embodiment 3, wherein the sealable opening 7350 of the mobile storage means 7300 is covered by at least one cover element 7352, 7354, wherein
   the corresponding sealable opening 7250 of the assembly room 7200 is covered by at least one corresponding cover element 7252, 7254, and wherein
   the at least one cover element and the at least one corresponding cover element are configured to couple with each other to open jointly.
Embodiment 5. The system 5000, 6000, 8000 of any one of embodiments 3 or 4, further comprising a sealing frame 7600, the sealing frame being configured to frame the sealable opening of the mobile storage means and/or the corresponding sealable opening of the assembly room.
Embodiment 6. The system 5000, 6000, 8000 of any one of embodiments 3 to 5, further comprising a UV-light source 7700, the UV-light source being preferably associated with the sealable opening of the mobile storage means and/or the corresponding sealable opening of the assembly room.
Embodiment 7. The system 5000, 6000, 8000 of any preceding embodiment, wherein the at least one manipulator 5100, 8100 is adapted to remove components from the mobile storage means and to assemble a smart tank within the assembly room from the removed components to form an assembled smart tank, wherein optionally the manipulator is further adapted to assemble at least one of the following, a filter, a valve and/or a sensor, to the assembled smart tank.
Embodiment 8. The system 5000, 6000, 8000 of any preceding embodiment, wherein the system includes components of at least one modular smart tank 1, the components include
   a top plate element 100, at least one sidewall element 200, and a bottom plate element 300, wherein
   the top plate element, the at least one sidewall element and the bottom plate element can be assembled to form a reservoir 500 of the smart tank for receiving at least one biochemical medium, wherein at least one of the top plate element, the at least one sidewall element and/or the bottom plate element comprises at least one channel 20, for guiding the at least one biochemical medium and/or an operating medium.
Embodiment 9. The system 5000, 6000, 8000 of any one of embodiment 8, wherein the assembled smart tank comprises at least one connector means for interconnecting the assembled smart tank with a further smart tank, wherein the connector means may provide a fluidical connection, and wherein the at least one manipulator is adapted to interconnect an assembled smart tank with at least one further assembled smart tank.
Embodiment 10. The system 5000, 6000, 8000 of any preceding embodiment, further comprising at least one mobile delivery means 5900, 8900, such as an automated guided vehicle, the mobile delivery means being adapted to transport the at least one mobile storage means 5300 to the assembly room, and wherein optionally the at least one mobile delivery means is adapted to transport mobile storage means to the assembly room in a predefined order.
Embodiment 11. The system 5000, 6000, 8000 of any preceding embodiment, wherein the assembly room is an assembly bag, comprising flexible wall elements, the assembly bag being optionally a sterile bag, that is further optionally a single-use bag.
Embodiment 12. The system 5000, 6000, 8000 of any preceding embodiment, wherein the manipulator is provided within the assembly room, and wherein the manipulator is optionally at least partially covered by a manipulator bag.
Embodiment 13. The system 5000, 6000, 8000 of any one of embodiments 1 to 12, wherein the manipulator is provided outside the assembly room, and wherein optionally the manipulator is at least partially covered by a manipulator bag, further optionally the manipulator bag being integrally formed with the assembly bag.
Embodiment 14. The system 5000, 6000, 8000 of any preceding embodiment, the system further comprising at least one clean room bag 6500, wherein the clean room bag is configured to receive at least one assembled smart tank 1 from the assembly room 6200, and optionally, the clean room bag is configured to receive multiple interconnected smart tanks from the assembly bag, wherein the at least one clean room bag is adapted to sealingly couple to the assembly room.
Embodiment 15. The system 5000, 6000, 8000 of embodiment 14, wherein the clean room bag 6500 is a sterile bag and optionally a single-use bag.
Embodiment 16. The system 5000, 6000, 8000 of any one of embodiments 11 to 15, wherein the assembly bag 6200 and/or the clean room bag 6500 is held by a support structure, and/or wherein the assembly bag and/or the clean room bag can be inflated.
Embodiment 17. The system 5000, 6000, 8000 of any one of embodiments 14 to 16, further comprising an adaptor plate element, the adaptor plate element is adapted to be arranged outside the clean room bag and to be coupled to an assembled smart tank, to cover a port of the smart tank and/or a filter at least partially, so as to give access to the smart tank from the outside of the clean room bag.
Embodiment 18. The system 5000, 6000, 8000 of any one of embodiments 14 to 17, wherein the clean room bag 6500 comprises at least one rail for guiding the at least one received assembled smart tank within the clean room bag.
Embodiment 19. The system 5000, 6000, 8000 of any preceding embodiment, wherein the mobile storage means 5300, 6300 comprises a tracking means, that allows geographical and/or local tracking of the mobile storage means.
Embodiment 20. The system 5000, 6000, 8000 of any preceding embodiment, wherein the mobile storage means 5300, 6300 is adapted to allow sterilization of smart tank components being stored within the mobile storage means.
Embodiment 21. The system 5000, 6000, 8000 of any preceding embodiment, wherein the mobile storage means 5300, 6300 is adapted to maintain the sterilized state of smart tank components being stored within the mobile storage means.
Embodiment 22. The system 5000, 6000, 8000 of any preceding embodiment, wherein the mobile storage means 5300, 6300 includes
   a sterile indicator, the sterile indicator being adapted to indicate whether the components stored in the mobile storage means were sterilized within the mobile storage means, and/or the mobile storage means including
   a port for receiving sterilized air allowing to perform sterile leakage testing of the mobile storage means.
Embodiment 23. The system 5000, 6000, 8000 of any preceding embodiment, wherein the mobile storage means 5300, 6300 includes
   a transport bag, that is preferably a sterile bag, the transport bag houses the smart tank components being stored within the mobile storage means, wherein optionally the transport bag is an assembly bag 8200.
Embodiment 24. The system 5000, 6000, 8000 of any preceding embodiment, wherein the mobile storage means 5300, 6300 includes at least one locking element, that is adapted to lock the mobile storage means during sterilization and/or transportation.
Embodiment 25. The system 5000, 6000, 8000 of any preceding embodiment, wherein the manipulator 5100, 8100 is adapted to disassemble smart tanks and optionally to remove assembled smart tanks from the clean room bag.
Embodiment 26. The system 5000, 6000, 8000 of any preceding embodiment, further comprising at least one recycling means, adapted for receiving disassembled components of a smart tank and preferably for shredding said components, wherein the recycling means may be adapted to sealingly couple to the assembly room, the recycling means optionally comprises
   at least one sealable opening that is configured for receiving disassembled components from the assembly room, wherein
   the sealable opening of the recycling means and the corresponding sealable opening of the assembly room are configured to open jointly, when the assembly room is sealingly coupled to the recycling means, and wherein the
   sealable opening of the recycling means is further optionally covered by at least one cover element, wherein
   the at least one cover element of the recycling means and the at least one corresponding cover element of the assembly room are configured to couple with each other to open jointly.
Embodiment 27. A method 10000 for assembling a modular smart tank for a biopharma process, the method comprising the following steps:
   providing 10100 at least one mobile storage means that stores components of at least one smart tank,
   providing 10200 an assembly room that is adapted to provide clean room conditions for assembling the modular smart tank, wherein the assembly room may be an assembly bag,
   providing 10300 a manipulator, and
   automatically assembling 10400 the smart tank within the assembly room from the components stored in the mobile storage means, using the manipulator.
Embodiment 28. The method 10000 of embodiment 27, further comprising providing a clean room bag,
   coupling said clean room bag with the assembly room, interconnecting an assembled smart tank with a further assembled smart tank, and
   moving the interconnected smart tanks from the assembly room to the clean room bag.
Embodiment 29. A mobile storage means 5300, 6300 for being used in a system according to any one of embodiments 1 to 26, the mobile storage means 5300, 6300, 7300 being adapted for storing components of at least one smart tank, further, the mobile storage means being adapted to sealingly couple to the assembly room 7200 of the system, the mobile storage means including:
   at least one sealable opening 7350 that is configured for allowing removal of components of the at least one smart tank from the mobile storage means, wherein the sealable opening 7350 of the mobile storage means is configured to open jointly with a corresponding sealable opening 7250 of an assembly room 7200 of the system, when the assembly room is sealingly coupled to the mobile storage means,
   the sealable opening 7350 of the mobile storage means being optionally covered by at least one cover element 7352, 7354, the at least one cover element of the sealable opening of the mobile storage means being configured to couple with a corresponding cover element 7252, 7254 of the corresponding sealable opening of the assembly room so that the sealable opening of the mobile storage means and the corresponding sealable opening of the assembly room open jointly.
Embodiment 30. The mobile storage means 5300, 6300 according to embodiment 30, being adapted as specified in any one of embodiments 19 to 24.
Embodiment 31. An assembly bag 6200, 7200, 8200, for being used in a system according to any one of embodiments 11 to 26, the assembly bag is adapted to provide clean room conditions for assembling at least one smart tank from components stored in a mobile storage means,
   the assembly bag 7200 comprises at least one corresponding sealable opening 7250 that is configured for receiving components from a mobile storage means, wherein the corresponding sealable opening of the assembly bag is configured to open jointly with a sealable opening 7350 of a mobile storage means, when the assembly bag is sealingly coupled to the mobile storage means,
   the corresponding sealable opening 7250 of the assembly bag being optionally covered by at least one corresponding cover element 7252, 7254, the at least one corresponding cover element of the corresponding sealable opening of the assembly bag being configured to couple with a cover element of the sealable opening of the mobile storage means so that the sealable opening of the mobile storage means and the corresponding sealable opening of the assembly room open jointly.
Embodiment 32. The assembly bag 6200, 7200, 8200, according to embodiment 31, being adapted as specified in any one of embodiments 11 or 16.
Embodiment 33. The assembly bag 6200, 7200, 8200 of any one of embodiments 31 or 32, wherein the assembly bag comprises a multi-ply outer shell, wherein a first ply of the shell is the outermost ply, and wherein a second ply of the shell is the innermost ply, and wherein the space surrounded by the second ply of the shell is pressurized with a pressure being lower than a pressure provided between the first and the second ply of the shell, wherein a colored gas may be provided between the first and the second ply of the shell.
Embodiment 34. The assembly bag 6200, 7200, 8200 of any one of embodiments 31 to 33, wherein the assembly bag is initially stored in a frame assembly 9100, the frame assembly comprising air-permeable wall elements 9110, 9120, and wherein the assembly bag comprises an air inlet port that serves for pressurizing the stored assembly bag for leakage testing.

## Claims

1. An assembly system (5000, 6000, 8000) for assembling a modular smart tank (1) for a bio-pharma process under clean room conditions, the system comprising:
at least one mobile storage means (5300, 6300) for storing components of at least one smart tank (1),
an assembly room (6200, 8200), wherein the assembly room is adapted to provide clean room conditions and wherein the assembly room is configured to allow assembly of at least one smart tank within the assembly room from the components stored in the mobile storage means, and
a manipulator (5100, 8100), being adapted to automatically assemble the smart tank within the assembly room.

2. The system (5000, 6000, 8000) of claim 1, wherein the mobile storage means (7300) includes a sealable opening (7350) that is configured for allowing removal of components of the at least one smart tank from the mobile storage means, and wherein
the assembly room (7200) comprises at least one corresponding sealable opening (7250) that is configured for receiving components from a mobile storage means, wherein
the sealable opening of the mobile storage means and the corresponding sealable opening of the assembly room are configured to open jointly, when the assembly room is sealingly coupled to the mobile storage means., wherein optionally the sealable opening (7350) of the mobile storage means (7300) is covered by at least one cover element (7352, 7354), wherein
the corresponding sealable opening (7250) of the assembly room (7200) is covered by at least one corresponding cover element (7252, 7254), and wherein
the at least one cover element and the at least one corresponding cover element are configured to couple with each other to open jointly wherein further optionally the system further comprises a sealing frame (7600), the sealing frame being configured to frame the sealable opening of the mobile storage means and/or the corresponding sealable opening of the assembly room.

3. The system (5000, 6000, 8000) of claim 2, further comprising a UV-light source (7700), the UV-light source being preferably associated with the sealable opening of the mobile storage means and/or the corresponding sealable opening of the assembly room.

4. The system (5000, 6000, 8000) of any preceding claim, wherein the at least one manipulator (5100, 8100) is adapted to remove components from the mobile storage means and to assemble a smart tank within the assembly room from the removed components to form an assembled smart tank, wherein optionally the manipulator is further adapted to assemble at least one of the following, a filter, a valve and/or a sensor, to the assembled smart tank.

5. The system (5000, 6000, 8000) of any preceding claim, wherein the system includes components of at least one modular smart tank (1), the components include
a top plate element (100), at least one sidewall element (200), and a bottom plate element (300), wherein
the top plate element, the at least one sidewall element and the bottom plate element can be assembled to form a reservoir (500) of the smart tank for receiving at least one biochemical medium, wherein at least one of the top plate element, the at least one sidewall element and/or the bottom plate element comprises at least one channel (20), for guiding the at least one biochemical medium and/or an operating medium.

6. The system (5000, 6000, 8000) of any preceding claim, further comprising at least one mobile delivery means (5900, 8900), such as an automated guided vehicle, the mobile delivery means being adapted to transport the at least one mobile storage means (5300) to the assembly room, and wherein optionally the at least one mobile delivery means is adapted to transport mobile storage means to the assembly room in a predefined order.

7. The system (5000, 6000, 8000) of any preceding claim, wherein the assembly room is an assembly bag, comprising flexible wall elements, the assembly bag being optionally a sterile bag, that is further optionally a single-use bag.

8. The system (5000, 6000, 8000) of any one of claims 1 to 7, wherein the manipulator is provided outside the assembly room, and wherein optionally the manipulator is at least partially covered by a manipulator bag, further optionally the manipulator bag being integrally formed with the assembly bag.

9. The system (5000, 6000, 8000) of any preceding claim, wherein the mobile storage means (5300, 6300) comprises a tracking means, that allows geographical and/or local tracking of the mobile storage means, and/or, wherein the mobile storage means (5300, 6300) is adapted to allow sterilization of smart tank components being stored within the mobile storage means, wherein the mobile storage means (5300, 6300) optionally includes
a transport bag, that is preferably a sterile bag, the transport bag houses the smart tank components being stored within the mobile storage means, wherein optionally the transport bag is an assembly bag (8200).

10. The system (5000, 6000, 8000) of any preceding claim, wherein the manipulator (5100, 8100) is adapted to disassemble smart tanks and optionally to remove assembled smart tanks from the clean room bag, and wherein the system optionally, further comprises at least one recycling means, adapted for receiving disassembled components of a smart tank and preferably for shredding said components, wherein the recycling means may be adapted to sealingly couple to the assembly room, the recycling means optionally comprises
at least one sealable opening that is configured for receiving disassembled components from the assembly room, wherein
the sealable opening of the recycling means and the corresponding sealable opening of the assembly room are configured to open jointly, when the assembly room is sealingly coupled to the recycling means, and wherein the
sealable opening of the recycling means is further optionally covered by at least one cover element, wherein
the at least one cover element of the recycling means and the at least one corresponding cover element of the assembly room are configured to couple with each other to open jointly.

11. A method (10000) for assembling a modular smart tank for a bio-pharma process, the method comprising the following steps:
providing (10100) at least one mobile storage means that stores components of at least one smart tank,
providing (10200) an assembly room that is adapted to provide clean room conditions for assembling the modular smart tank, wherein the assembly room may be an assembly bag,
providing (10300) a manipulator, and
automatically assembling (10400) the smart tank within the assembly room from the components stored in the mobile storage means, using the manipulator, the method (10000) optionally, further comprising
providing a clean room bag,
coupling said clean room bag with the assembly room, interconnecting an assembled smart tank with a further assembled smart tank, and
moving the interconnected smart tanks from the assembly room to the clean room bag.

12. A mobile storage means (5300, 6300) for being used in a system according to any one of claims 1 to 10, the mobile storage means (5300, 6300, 7300) being adapted for storing components of at least one smart tank, further, the mobile storage means being adapted to sealingly couple to the assembly room (7200) of the system, the mobile storage means including:
at least one sealable opening (7350) that is configured for allowing removal of components of the at least one smart tank from the mobile storage means, wherein the sealable opening (7350) of the mobile storage means is configured to open jointly with a corresponding sealable opening (7250) of an assembly room (7200) of the system, when the assembly room is sealingly coupled to the mobile storage means,
the sealable opening (7350) of the mobile storage means being optionally covered by at least one cover element (7352, 7354), the at least one cover element of the sealable opening of the mobile storage means being configured to couple with a corresponding cover element (7252, 7254) of the corresponding sealable opening of the assembly room so that the sealable opening of the mobile storage means and the corresponding sealable opening of the assembly room open jointly.

13. The mobile storage means (5300, 6300) according to claim 12, wherein the mobile storage means (5300, 6300) comprises a tracking means, that allows geographical and/or local tracking of the mobile storage means, and/or, wherein the mobile storage means (5300, 6300) is adapted to allow sterilization of smart tank components being stored within the mobile storage means, wherein the mobile storage means (5300, 6300) optionally includes
a transport bag, that is preferably a sterile bag, the transport bag houses the smart tank components being stored within the mobile storage means, wherein optionally the transport bag is an assembly bag (8200).

14. An assembly bag (6200, 7200, 8200), for being used in a system according to any one of claims 7 to 10, the assembly bag is adapted to provide clean room conditions for assembling at least one smart tank from components stored in a mobile storage means,
the assembly bag (7200) comprises at least one corresponding sealable opening (7250) that is configured for receiving components from a mobile storage means, wherein the corresponding sealable opening of the assembly bag is configured to open jointly with a sealable opening (7350) of a mobile storage means, when the assembly bag is sealingly coupled to the mobile storage means,
the corresponding sealable opening (7250) of the assembly bag being optionally covered by at least one corresponding cover element (7252, 7254), the at least one corresponding cover element of the corresponding sealable opening of the assembly bag being configured to couple with a cover element of the sealable opening of the mobile storage means so that the sealable opening of the mobile storage means and the corresponding sealable opening of the assembly room open jointly.

15. The assembly bag (6200, 7200, 8200), according to claim 14, being adapted as specified in any one of claims 7 or 8, wherein
the assembly bag optionally comprises a multi-ply outer shell, wherein a first ply of the shell is the outermost ply, and wherein a second ply of the shell is the innermost ply, and wherein the space surrounded by the second ply of the shell is pressurized with a pressure being lower than a pressure provided between the first and the second ply of the shell, wherein a colored gas may be provided between the first and the second ply of the shell, and/or wherein
the assembly bag is initially stored in a frame assembly (9100), the frame assembly comprising air-permeable wall elements (9110, 9120), and wherein the assembly bag comprises an air inlet port that serves for pressurizing the stored assembly bag for leakage testing.
